(19) Europäisches Patentamt
European Patent Office
Office européen des brevets

(11) EP 1 406 910 B1

(12) # FASCICULE DE BREVET EUROPEEN

(45) Date de publication et mention de la délivrance du brevet:
**13.01.2010 Bulletin 2010/02**

(21) Numéro de dépôt: **02764988.8**

(22) Date de dépôt: **16.07.2002**

(51) Int Cl.:
***C07F 9/40*** *(2006.01)* ***C07F 9/38*** *(2006.01)*
***A61K 31/663*** *(2006.01)* ***A61P 19/08*** *(2006.01)*
***A61P 35/00*** *(2006.01)*

(86) Numéro de dépôt international:
**PCT/FR2002/002526**

(87) Numéro de publication internationale:
**WO 2003/008425 (30.01.2003 Gazette 2003/05)**

(54) **PROCEDES DE PREPARATION DE DERIVES DE BISPHOSPHONATES**
HERSTELLUNGSVERFAHREN VON DERIVATE VON BISPHOSPHONATEN
PREPARATION METHODS OF DERIVATIVES OF BISPHOSPHONATES

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **16.07.2001 FR 0109483**
**21.03.2002 FR 0203540**

(43) Date de publication de la demande:
**14.04.2004 Bulletin 2004/16**

(73) Titulaire: **Universite Paris 13**
**93430 Villetaneuse (FR)**

(72) Inventeurs:
- **LECOUVEY, Marc**
**F-75017 Paris (FR)**
- **LEROUX, Yves**
**F-75013 Paris (FR)**
- **KRAEMER, Michel**
**F-75012 Paris (FR)**
- **CREPIN, Michel**
**F-75013 Paris (FR)**
- **EL MANOUNI, Driss**
**F-75013 Paris (FR)**
- **LOURIKI, Malika,**
**Résidence Beau Lieu**
**CASABLANCA (MA)**

(74) Mandataire: **Tezier Herman, Béatrice et al**
**Cabinet Becker & Associés**
**25, rue Louis Le Grand**
**75002 Paris (FR)**

(56) Documents cités:
**EP-A- 0 416 689** **EP-A- 0 891 979**
**WO-A-00/12517** **WO-A-00/71104**
**WO-A-01/15703** **WO-A-01/30788**
**WO-A-01/39724** **WO-A-90/13704**
**WO-A-92/11267** **WO-A-92/11269**
**WO-A-93/00348** **WO-A-96/39107**
**WO-A-99/20635** **WO-A-99/51613**
**DE-A- 3 700 772** **GB-A- 2 082 585**
**GB-A- 2 113 688** **US-A- 4 440 646**

- **NIEMI, R. ET AL: "Bisphosphonate prodrugs: synthesis and in vitro evaluation of alkyl and acyloxymethyl esters of etidronic acid as bioreversible prodrugs of etidronate" EUROPEAN JOURNAL OF PHARMACEUTICAL SCIENCES (2000), 11(2), 173-180 , - août 2000 (2000-08) XP001126281**
- **NESTEROV L.V. ET AL.: "Alkyl trimethyl- silyl (alpha-(trimethylsiloxy)alkyl) phosphonates and products of their desilylation" JOURNAL OF GENERAL CHEMISTRY USSR., vol. 54, no. 1, - 20 juin 1984 (1984-06-20) pages 40-42, XP002220399 CONSULTANTS BUREAU. NEW YORK., US**
- **TURHANEN, PETRI A. ET AL: "Bisphosphonate prodrugs. Selective synthesis of (1-hydroxyethylidene)-1,1-bisphosphonate partial esters" SYNTHESIS (2001), (4), 633-637 , 2001, XP002220400**
- **GELDER VAN J M ET AL: "ANTICALCIFICATION AND ANTIRESORPTION EFFECTS OF BISACYLPHOSPHONATES" BONE, PERGAMON PRESS., OXFORD, GB, vol. 16, no. 5, 1 mai 1995 (1995-05-01), pages 511-520, XP000615577 ISSN: 8756-3282**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- CHEMICAL ABSTRACTS, vol. 129, no. 12, 21 septembre 1998 (1998-09-21) Columbus, Ohio, US; abstract no. 148722, CHEN, WEIPING ET AL: "Study on property and acid dissociation constant of.alpha.-hydroxyethylidene-bis(O-alkyl phosphonic acid)" XP002220402 & XIANGTAN DAXUE ZIRAN KEXUE XUEBAO (1997), 19(2), 62-64, 67 , 1997,
- GUMIENNA-KONTECKA E. ET AL.: "Bisphosphonate chelating agents." JOURNAL OF INORGANIC BIOCHEMISTRY, vol. 89, no. 1-2, - 10 avril 2002 (2002-04-10) pages 13-17, XP002220401 NEW YORK, NY, US
- MARTIN M.B. ET AL.: "Bisphosphonates Inhibit the Growth of Trypanosoma brucei, Trypanosoma cruzi, Leishmania donovani, Toxoplasma gondii, and Plasmodium falciparum: A Potential Route to Chemotherapy" JOURNAL OF MEDICINAL CHEMISTRY (2001), 44(6), 909-916 , 15 mars 2001 (2001-03-15), XP002220934 & J. MED. CHEM. SUPPORTING INFORMATION, [en ligne] Extrait de l'Internet: <URL:http://pubs.acs.org/subscribe/journal s/jmcmar/suppinfo/jm0002578/jm0002578_s.pd f> [extrait le 2002-11-15]
- OSTOVIC, DRAZEN ET AL: "Development of subcutaneous and intramuscular formulations of calcium alendronate salts" DRUG DEVELOPMENT AND INDUSTRIAL PHARMACY (1995), 21(10), 1157-69 , 1995, XP008010717
- SORBERA, L. A. ET AL: "Zoledronate disodium: treatment of tumor-induced hypercalcemia angiogenesis inhibitor" DRUGS OF THE FUTURE (2000), 25(3), 259-268 , 2000, XP008010716
- JAGDEV S P ET AL: "THE BISPHOSPHONATE, ZOLEDRONIC ACID, INDUCES APOPTOSIS OF BREAST CANCER CELLS: EVIDENCE FOR SYNERGY WITH PACLITAXEL" BRITISH JOURNAL OF CANCER, LONDON, GB, vol. 84, no. 8, 2001, pages 1126-1134, XP008001603 ISSN: 0007-0920
- LEE M V ET AL: "BISPHOSPHONATE TREATMENT INHIBITS THE GROWTH OF PROSTATE CANCER CELLS" CANCER RESEARCH, AMERICAN ASSOCIATION FOR CANCER RESEARCH, BALTIMORE, MD, US, vol. 61, 15 mars 2001 (2001-03-15), pages 2602-2603, XP001064448 ISSN: 0008-5472
- HIRAGA T ET AL: "THE BISPHOSPHONATE IBANDRONATE PROMOTES APOPTOSIS IN MDA-MB-231 HUMAN BREAST CANCER CELLS IN BONE METASTASES" CANCER RESEARCH, AMERICAN ASSOCIATION FOR CANCER RESEARCH, BALTIMORE, MD, US, vol. 61, 1 juin 2001 (2001-06-01), pages 4418-4424, XP001064447 ISSN: 0008-5472


Remarques:

Le dossier contient des informations techniques présentées postérieurement au dépôt de la demande et ne figurant pas dans le présent fascicule.

## Description

La présente invention concerne des procédés de préparation de dérivés partiellement estérifiés de l'acide 1-hydroxyméthylène-1,1-bisphosphonique (ou bisphosphonate). Elle décrit également les dérivés ainsi préparés, des compositions pharmaceutiques les comprenant, leur application en thérapeutique, notamment pour le traitement des tumeurs cancéreuses et des maladies virales ou inflammatoires hépatiques.

Les dérivés des acides 1-hydroxyméthylène-1,1-biphosphoniques présentent des propriétés anti-tumorales remarquables et leurs applications médicales ont fait l'objet de recherches approfondies. Ces dérivés, qui sont caractérisés par une liaison P-C-P, sont des analogues stables de pyrophosphate et sont résistants à l'hydrolyse enzymatique. En ce qui concerne les applications des dérivés d'acide diphosphonique en thérapeutique, on sait que divers dérivés ont des propriétés utiles dans le traitement de l'inflammation, de l'ostéoporose, ou de certaines métastases osseuses. En particulier, ils sont utilisés grâce à leurs propriétés inhibitrices de la résorption osseuse pour traiter de nombreuses maladies caractérisées par un métabolisme anormal du calcium. La résorption osseuse est accrue de manière pathologique dans les métastases de certains cancers, tels que cancers du sein ou de la prostate, et de façon accélérée dans les différents types d'ostéoporose dont celle liée à l'âge. Les patients présentant ce type de métastases peuvent actuellement bénéficier de protocoles de traitements incluant des bisphosphonates (Diel et al., 2000 ; Lipton, 2000 ; Mincey et al., 2000). Il est à souligner que le système osseux est le troisième site commun de métastases et que plus de 80% des patients décédés d'un cancer présentent des tumeurs osseuses à l'autopsie.

De nombreux dérivés d'acide diphosphonique ont été décrits dans la littérature ainsi que leurs propriétés utiles dans divers domaines d'application.

Ainsi, par exemple, l'acide didronique est bien connu depuis des années comme principe actif de médicament pour le traitement des maladies osseuses telles que l'ostéoporose, et plus particulièrement l'étidronate disodique, décrit dans le brevet FR 8.441 M. Une structure dérivée est décrite par exemple dans le brevet US 4,705,651 relatif à l'acide alendronique possédant des propriétés d'inhibition de la perte osseuse, permettant son utilisation également dans le traitement de l'ostéoporose. Une autre structure dérivée, l'acide ibandronique présentant des propriétés anti-inflammatoires, est décrite dans le brevet GB 2.312.165.

Des dérivés d'acide alcane-1,1-diphosphonique résultant du couplage d'un acide diphosphonique avec un acide aminé, et présentant une activité antitumorale et d'inhibition de la résorption osseuse, ont été décrits dans la demande de brevet WO 97 49711. D'autres dérivés d'acide diphosphonique, comportant un substituant phényle en position 1, sont connus pour leur activité anti-inflammatoire comme dans le brevet US 4 473 560 relatif à des dérivés à activité anti-inflammatoire, notamment anti-arthritique, ou dans la demande de brevet WO 97 04785 décrivant des diphosphonates substitués par un groupe phénolique présentant des propriétés anti-néoplasiques. On a aussi décrit dans le brevet EP 0 537 008 des diphosphonates comportant un groupe lipophile utiles pour la préparation d'un médicament inhibiteur de protéine prényl transférase susceptible de bloquer la transformation néoplasique provoquée par des oncogènes ras.

L'action anti-ostéoclastique des bisphosphonates se ferait par induction de l'apoptose dans les ostéoclastes (Luckman et al., 1998) via une inhibition de la voie du mévalonate et de la synthèse du cholestérol.

Les bisphosphonates inhibent, *in vitro,* la prolifération de cellules tumorales mammaires (Fromigue et al., 2000 ; Hiraga et al., 2001 ; Jagdev et al., 2001 ; Senaratne et al., 2000 ; Yoneda et al., 2000) et de cellules tumorales de prostate (Lee et al., 2001). Cette inhibition *in vitro* est due à l'apoptose des cellules tumorales et s'accompagne d'une réduction de l'expression du gène *bcl-2* (Senaratne et al., 2000) et d'une activation de caspases (Fromigue et al., 2000).

A côté des effets précédents, les bisphosphonates peuvent avoir plusieurs actions complémentaires, telle l'inhibition de l'adhésion, *in vitro*, de cellules tumorales mammaires à des lamelles osseuses (Boissier et al., 1997 ; Van der Pluijm et al., 1996), l'induction *in vitro* de l'apoptose de cellules de myélomes (Shipman et al., 1997, 1998, 2000a) ou l'inhibition de l'activité (mais non pas de la production) de métalloprotéases matricielles par des cellules de carcinomes mammaires ou de prostate (Boissier et al., 2000 ; Ichinose et al., 2000 ; Teronen et al., 1997).

Les bisphosphonates ont également été utilisés dans le traitement de leucémies lymphoblastiques (Ogihara et al., 1995 ; Takagi et al., 1998). Les cellules leucémiques induisent une angiogenèse dans la moelle osseuse, angiogenèse nécessaire à leur prolifération. Le traitement des leucémies lymphoblastiques par les bisphosphonates s'accompagnent d'une baisse importante de l'angiogenèse (Perez-Atayde et al., 1997).

Des données récentes soulignent l'implication très probable de facteurs angiogéniques dans le développement de métastases osseuses. Ainsi, Van der Pluijm et al. (2001), dans un travail utilisant un modèle murin *in vivo* de métastases expérimentales de cellules mammaires (MDA MB 231), ont émis l'hypothèse que l'augmentation de l'expression de facteurs angiogénique (VEGF) et ostéolityque (PTH) par les cellules tumorales serait impliquée dans l'ostéotropisme et l'ostéolyse des métastases osseuses.

Si à l'heure actuelle, l'activité anti proliférative des bisphosphonates sur les cellules tumorales *in vitro* est bien établie, le fait que les bisphosphonates présenteraient une action anti tumorale *in vivo* est une question encore ouverte. Quelques données sembleraient en faveur d'une action anti proliférative des bisphosphonates, *in vivo,* au niveau des

foyers métastatiques osseux. Ainsi, selon Hiraga et al. (2001), les bisphosphonates feraient diminuer la charge tumorale des cellules métastatiques des os. Cependant, aucune donnée concernant une action anti tumorale des bisphosphonates *in vivo* sur les tumeurs primaires n'a été, à notre connaissance, rapportée.

Le métabolisme des bisphosphonates par l'organisme est faible et la fraction active des produits ne représenterait que 3 à 7 % de la quantité absorbée. Cette faible biodisponibilité des bisphosphonates par voie orale résulte de leur faible lipophilie (Lin, 1996) qui est due à leur haute ionisation au pH physiologique. Leur absorption est encore réduite en raison de la forte charge négative et de la taille relativement importante de ces molécules (Ruifrok and Mol, 1983 ; Pade and Stavchanvsky, 1997). De plus, l'absorption des bisphosphonates est encore réduite par leur forte complexation avec le calcium et les autres ions divalents dans l'espace intestinal (Lin, 1996). Leur administration est souvent associée à des troubles gastriques et à d'autres effets secondaires (Adami and Zamberlan., 1996 ; Mondelo et al., 1997).

En outre, la résistance acquise aux médicaments est une importante difficulté rencontrée aujourd'hui dans le traitement du cancer et la plupart des cancers chez l'homme sont résistants aux effets des chimiothérapies.

Afin d'améliorer leurs effets thérapeutiques, différentes solutions ont été proposées. La première consiste en l'utilisation d'un vecteur peptidique greffé sur la chaîne latérale de l'acide hydroxybisphosphonique (Ezra et al., 2000). D'autres études suggèrent d'encapsuler la drogue dans des microsphères (Patashnik et al., 1997) ou dans des liposomes (Ylitalo et al., 1998).

La présente invention divulgue des dérivés de bisphosphonates présentant une biodisponibilité améliorée et une efficacité thérapeutique satisfaisante.

Deux procédés de synthèse décrits dans la littérature permettent l'obtention des acides 1-hydroxyméthylène 1,1-bisphosphoniques.

Le premier permet d'accéder aux produits désirés en une seule étape. Il consiste à chauffer un mélange d'acide carboxylique en présence d'acide phosphoreux et de trichlorure de phosphore à 100°C pendant plusieurs heures.

Les conditions de cette réaction ont été très étudiées. En effet, depuis 1970, plus de cinquante brevets et articles ont été publiés. Les inconvénients de cette méthode sont cependant nombreux. En effet, les conditions opératoires drastiques ne sont pas adaptées à des substrats fragiles. De plus, l'extraction du bisphosphonate du milieu réactionnel est souvent délicate à mettre en oeuvre.

La seconde procédure est une méthode indirecte qui passe par la synthèse d'esters 1-hydroxyméthylène-1,1-bisphosphoniques suivie d'une étape de déalkylation. Les α-cétophosphonates sont couramment préparés suivant la réaction de Michaelis Arbuzov, à partir d'un phosphite de structure $P(OR)_3$ et d'un chlorure d'acide. L'ester bisphosphonique est ensuite normalement obtenu par une réaction de l'α-cétophosphonate avec un dialkylphosphite $HOP(OR)_2$.

L'étape de déalkylation est réalisée soit par hydrolyse en milieu acide chlorhydrique soit par un traitement au bromotriméthylsilane suivie d'une méthanolyse.

Malheureusement, ces deux procédés de synthèse ne permettent pas d'accéder à des acides 1-hydroxyméthylène-1,1-bisphosphoniques partiellement estérifiés.

La présente invention a donc pour but de proposer des procédés de préparation de bisphosphonates permettant d'introduire régiosélectivement une

ou plusieurs fonctions esters sur les groupements acides phosphoniques.

La présente invention décrit des dérivés de bisphosphonate de formule (1) suivante :

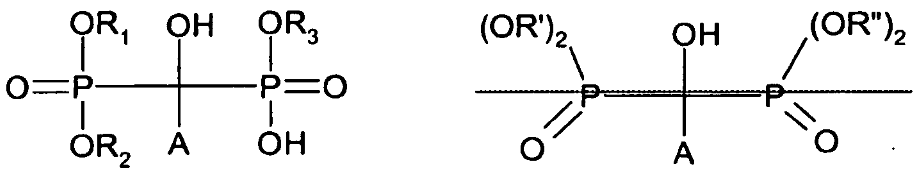

(I) (I')

dans laquelle $R_1$, $R_2$ et $R_3$, identiques ou différents, représentent un atome d'hydrogène, un radical alkyle, aryle, acyloxyalkyle ou hétérocycle,

A représente un radical de formule $-(CH_2)n-R_4$,

$R_4$ représente un atome d'hydrogène, un radical alkyle, un radical aryle, un hétérocycle ou un radical de formule $-NR_5R_6$,

$R_5$ et $R_6$, identiques ou différents, représentent un atome d'hydrogène ou un radical alkyle,

n est un entier de 0 à 24 inclusivement,

à l'exclusion des composés de formule (1) dans laquelle $R_1$, $R_2$ et $R_3$ représentent simultanément un atome d'hydrogène, leurs isomères optiques et géométriques, leurs racémates, leurs sels, leurs hydrates et leurs mélanges.

Ainsi, l'invention décrit des composés qui ont l'avantage d'être très biodisponibles, en particulier qui présentent une biodisponibilité améliorée par rapport à des composés décrits ou disponibles comme actifs thérapeutiques, tels que notamment l'Etidronate (Procter & Gamble), Alendronate (Merck), la Pamidronate (Novartis), Olpadronate (Gador), Ibandronate (Borhinger-Mannheim), le composé EB1053 (Leo), le composé YH 529 (Aventis) ou le Residronate (Procter & Gamble).

La présente invention décrit également une composition pharmaceutique comprenant dans un support pharmaceutiquement acceptable au moins un composé de formule (I) tel que décrit ci-dessus, éventuellement en association avec un autre actif thérapeutique.

Elle divulgue aussi l'utilisation d'au moins un composé de formule (I) pour la préparation d'une composition pharmaceutique destinée à traiter une maladie caractérisée par un métabolisme du calcium anormal, tel que des cancers ou l'ostéoporose.

Elle divulgue également l'utilisation d'au moins un composé de formule (I) pour la préparation d'une composition pharmaceutique destinée à traiter une maladie virale ou inflammatoire hépatique.

La présente invention a pour objet des procédés de préparation des composés de formule (I) tels que définis ci-dessus.

Les radicaux alkyle, aryle, hétérocycle et acyloxyalkyle précédemment cités sont éventuellement substitués par au moins un groupement choisi parmi un radical aryle, un radical hétérocycle, un radical hétérocycloalkyle, un radical alkyle, un radical alkényle, un radical alkynyle, un radical alkylthio, un atome d'halogène, de préférence Cl, F, Br, un radical hydroxyle, un groupement $NO_2$, un radical alkoxy, un groupe ester (-COOR), un groupe aminé -NRR'", une fonction acide, un groupement amide (-CONHR ou -NHCOR), où R et R'" représentent, indépendamment l'un de l'autre, un atome d'hydrogène, un radical alkyle, aryle, hétéroaryle ou acyloxyalkyle.

Selon la présente invention, le terme "alkyle" désigne plus particulièrement un radical hydrocarboné linéaire, ramifié ou cyclique ayant de 1 à 24, de préférence 1 à 12, atomes de carbone tels que méthyle, éthyle, propyle, isopropyle, butyle, isobutyle, *tert*-butyle, pentyle, néopentyle, n-hexyle. Les groupes en $C_1$-$C_6$ sont particulièrement préférés. Les groupes méthyle et éthyle sont tout particulièrement préférés.

Le terme « alkényle » se réfère à un radical hydrocarboné présentant au moins une insaturation éthylénique et le terme « alkynyle » se réfère à un radical hydrocarboné présentant au moins une insaturation acétylénique.

Les groupes « aryle » sont des systèmes hydrocarbonés aromatiques mono-, bi- ou tri-cycliques ayant de 6 à 18 atomes de carbone. On peut notamment citer, à titre d'exemples, un radical phenyl, $\alpha$-naphtyl, $\beta$-naphtyl ou antracényl.

Les groupes « alkoxy » correspondent aux groupes alkyle définis ci-dessus reliés au reste du composé par l'intermédiaire d'une liaison -O- (éther).

Les groupes « alkylthio » correspondent aux groupes alkyle définis ci-dessus reliés au reste du composé par l'intermédiaire d'une liaison -S- (thioéther).

Les radicaux « acyloxyalkyle » correspondent aux groupes acyloxy reliés au reste du composé par l'intermédiaire d'une chaîne alkylène (C1-24), de préférence de C1-4. On peut notamment citer le radical acyloxyméthyle ou pivaloyloxyméthyle.

Par « halogène », on entend un atome de fluor, de chlore, de brome ou d'iode.

Les groupes arylalkyle (ou aralkyle) sont des groupes comprenant un reste aryle tel que défini ci-dessus lié au reste du composé au moyen d'une chaîne alkylène.

Les groupes « hétérocycles » ou « hétérocycloalkyle » sont des groupes comportant de 5 à 18 maillons cycliques comprenant un ou plusieurs hétéroatomes (généralement N, O, S ou P), de préférence de 1 à 5 hétéroatomes endocycliques. Ils peuvent être mono-, bi- ou tri-cycliques. Ils peuvent être aromatiques ou non. Préférentiellement, et plus particulièrement pour $R_5$, il s'agit d'hétérocycles aromatiques. Des exemples de groupes hétérocycliques aromatiques sont les groupes pyridine, pyridazine, pyrimidine, pyrazine, furane, thiophene, pyrrole, oxazole, thiazole, isothiazole, imidazole, pyrazole, oxadiazole, triazole, thiadiazole et triazine. Des exemples de bicycles sont notamment les groupes quinoline, isoquinoline et quinazoline (pour deux cycles à 6 sommets) et indole, benzimidazole, benzoxazole, benzothiazole et indazole (pour un cycle à 6 sommets et un cycle à 5 sommets). Des hétérocycles non aromatiques sont notamment pipérazine, pipéridine, etc.

Parmi les atomes de métal alcalin, on peut notamment citer le sodium et le potassium. Parmi les alcalino-terreux, on peut notamment citer le calcium.

Les composés préférentiellement préparés selon l'invention présentent une formule (I) dans laquelle R1, R2 et R3 représentent un atome d'hydrogène ou un radical alkyle de C1-12, plus particulièrement de C1-6.

De manière avantageuse, au moins deux des substituants $R_1$, $R_2$ et $R_3$ sont différents d'un atome d'hydrogène.

Selon un mode particulièrement préféré de l'invention, les substituants $R_1$, $R_2$ et $R_3$, qui sont différents d'un atome d'hydrogène, sont identiques.

Les composés préférentiellement préparés selon l'invention présentent une formule (I) dans laquelle R1, R2

et R3 représentent le radical méthyle ou éthyle.

Les composés préférentiellement préparés selon l'invention présentent une formule (I) dans laquelle A représenté un radical de formule -(CH2)n-$R_4$, dans laquelle n est entier de 1 à 12 inclusivement, de préférence de 1 à 6 inclusivement, avantageusement de 1 à 3 inclusivement.

Les composés préférentiellement préparés selon l'invention présentent une formule (I) dans laquelle $R_4$ représente un radical alkyle C1-C6, un radical hétérocycle, un radical phényle, ou un radical de formule -$NR_5R_6$, dans laquelle $R_5$ et $R_6$, identiques ou différents, représentent un atome d'hydrogène ou un radical alkyle de C1-6, dans laquelle avantageusement n est un entier de 1 à 6 inclusivement, de préférence de 1 à 3 inclusivement.

Selon un mode particulier de l'invention, les composés préférentiellement préparés selon l'invention présentent une formule (I) dans laquelle A représente un radical choisi parmi :

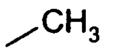 ; 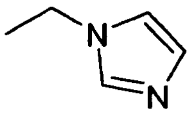 ; 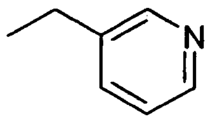 ; 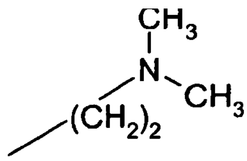 ;

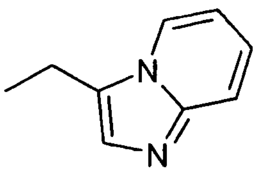 ; 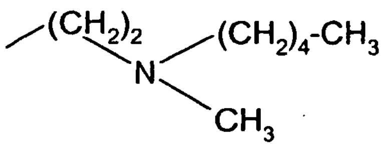 ; 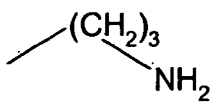 ;

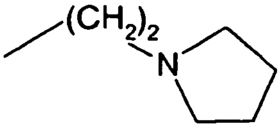 ; 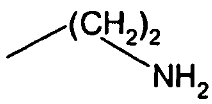 ; ou 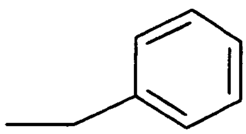

Selon un aspect particulier de l'invention, les composés de la présente invention présentent la formule (I) telle que définie ci-dessus, à l'exclusion des composés où A représente

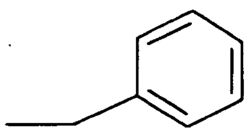,

R1 et R3 représentent un radical méthyle et R2 représente un atome d'hydrogène.

Les composés de formule (I) préparés selon l'invention, présentent une biodisponibilité avantageuse, voire améliorée par rapport à certains produits connus de l'état de la technique. Les composés selon l'invention présentent ainsi un effet thérapeutique très satisfaisant. En effet, sans vouloir se lier à une quelconque théorie de l'invention, les liaisons esters des composés de formule (I) selon l'invention augmentent leur lipophilie et par ce biais notamment les composés semblent mieux passer la barrière gastro-instestinale lorsqu'ils sont administrés par voie orale, les liaisons esters sont ensuite lysées pour libérer l'acide bisphosphonique correspondant.

La présente invention a donc pour objet des procédés de préparation de composés de formules (I).

Ces procédés de préparation présentent de nombreux avantages. Il sont simples à mettre en oeuvre industriellement et permettent d'obtenir un rendement élevé en bisphosphonates.

Le procédé général de préparation des composés de formule (I) de la présente invention comprend les étapes suivantes :

- la mise en contact d'au moins un halogénure d'acide de formule (II) : ACOX, ou d'un α-cétophosphonate de formule (III) :

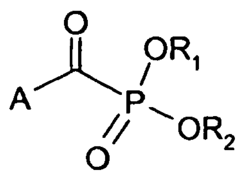

avec au moins un phosphite silylé de formule (IV) :

$$P[OSialk_3]_x[OR_3]_{3-x} \qquad (IV)$$

dans lesquelles $R_1$, $R_2$ et $R_3$, identiques ou différents, représentent un radical alkyle, aryle, acyloxyalkyle, ou hétérocycle,
A étant tel que défini ci-dessus,
X représente un atome d'halogène, de préférence le chlore,
alk représente un radical alkyle de C1-6,
x représente 2 ou 3,

- une hydrolyse des composés obtenus à l'étape précédente.

Le radical alkyle de C1-6 est tel que défini précédemment.

L'atome d'halogène peut être un atome de chlore, de brome, d'iode ou de fluor, de préférence X est l'atome de chlore.

Lorsque R1 et R2 sont différents de l'atome d'hydrogène dans les composés de formule (I), le procédé de préparation des composés de formule (I) comprend les étapes suivantes :

- la mise en contact d'au moins un halogénure d'acide de formule (II) : ACOX avec au moins un phosphite de formule (V) : P(OR1)(OR2)(OR), avec R1, R2 et R, identiques ou différents, représentent un radical alkyle, aryle, acyloxyalkyle, ou hétérocycle, pour former un α-cétophosphonate de formule (III),
- la mise en contact de l'α-cétophosphonate obtenu à l'étape précédente avec au moins un phosphite silylé de formule (IV) tel que défini ci-dessus,
- une hydrolyse des composés obtenus à l'étape précédente.

Plus précisément, lorsque R1, R2 et R3 sont différents de l'atome d'hydrogène, le phosphite silylé de formule (IV) de préférence mis oeuvre correspond à un composé de formule (IV) dans laquelle x vaut 2.

Plus précisément, lorsque R1 et R2 sont différents de l'atome d'hydrogène et R3 représente un atome d'hydrogène, le phosphite silylé de formule (IV) de préférence mis oeuvre correspond à un composé de formule (IV) dans laquelle x vaut 3.

Lorsque l'on souhaite obtenir des composés de formule (I) dans laquelle R1 et R3 sont méthyle et R2 représente un atome d'hydrogène, le procédé de préparation est mis en oeuvre par la mise en contact d'1 équivalent molaire d'un halogénure d'acide de formule (II) : ACOX ; avec deux équivalents-molaires d'au moins un phosphite silylé de formule $P[OSiMe_3]_2[OMe]$.

Le procédé de préparation des composés de formule (I) où R1 est différent de l'atome d'hydrogène et R2 et R3 représentent un atome d'hydrogène, est mis en oeuvre avec une mise en contact d'au moins un halogénure d'acide de formule (II) : ACOX ; avec au moins un phosphite silylé de formule (IV) dans laquelle x vaut 2 et une mise en contact du produit ainsi obtenu avec au moins un phosphite silylé de formule (IV) dans laquelle x vaut 3.

L'hydrolyse du procédé de préparation des composés de formule (I) s'effectue généralement dans un solvant capable de fournir des protons, tel que notamment le méthanol ou l'éthanol. De préférence, le solvant utilisé est le méthanol.

Les rendements obtenus en composés de formule (I) sont quasi-quantitatifs. En outre, ce procédé présente l'avantage de mettre en oeuvre *in situ* successivement les étapes mentionnées ci-dessus, sans nécessité de transférer des produits.

De manière générale, les étapes du procédé selon l'invention peuvent être réalisées à température ambiante (18-30°C) et à pression atmosphérique. Bien entendu, il est à la portée de l'homme du métier de varier ces conditions, si nécessaire.

Dans certains cas, les réactions avec le phosphite silylé peuvent être exothermiques, sans toutefois présenter de réactions secondaires. Ce caractère exothermique peut rendre nécessaire de mettre en oeuvre ces réactions à des températures plus basses que l'ambiante, plus particulièrement à des températures comprises entre -70°C et 20°C, notamment lorsque l'on met en oeuvre le chlorure de nitrobenzoyle.

Les étapes qui précèdent l'étape d'hydrolyse du procédé selon l'invention peuvent être mises en oeuvre en masse ou dans un solvant, tel que $CHCl_3$, THF, $CH_3CN$, $CH_2Cl_2$, ou l'éther. Avantageusement, elles sont mises en oeuvre en masse.

L'étape d'hydrolyse est réalisée avantageusement pendant 1 à 4 heures, plus particulièrement pendant environ deux heures. Elle est de préférence mise en oeuvre à température ambiante, entre 18 et 30°C.

Après l'étape d'hydrolyse, le procédé selon l'invention comprend de préférence la purification du composé de formule (I) obtenu. Cette purification est réalisée par tout moyen connu en soi. De préférence, on réalise une purification par au moins deux lavages successifs dans un solvant approprié, notamment l'éther.

Les produits de départ du procédé selon l'invention sont des produits commercialisés et sont donc facilement accessibles. Concernant les phosphites, le tris triméthylsilyl phosphite est commercial. Les autres phosphites peuvent être préparés selon le procédé suivant. Le dialkylphosphite est traité par une solution d'ammoniaque concentrée à 0 °C. Après évaporation de l'eau, le sel obtenu est traité par de l'hexaméthyldisilazane pendant 4 heures à reflux. Le schéma réactionnel est le suivant.

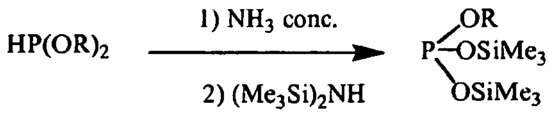


Des schémas réactionnels du procédé de préparation des composés de formule (I) selon l'invention sont représentés à la figure 1.

Conformément à la présente invention, l'acide phénylacétique bisphosphonique peut être préparé par un procédé analogue à la méthode de préparation de dérivés hydroxydiphosphoniques décrite dans le brevet FR 2 669 348 et par Y. Leroux et al., Phosphorus, Sulfur and Silicon, 63, 181 (1991). Le procédé consiste, dans une première étape, à faire réagir un chlorure d'acide de formule (II) ACOX, telle que définie ci-dessus, sur un mélange de diméthylphosphite et de triméthylphosphite, puis dans une deuxième étape, à hydrolyser les fonctions esters formées dans la première étape, par hydrolyse acide, suivie si nécessaire par une salification.

Le chlorure d'acide utilisé dans la première étape peut donc être le chlorure de phénylacétyle ($C_6H_5$-$CH_2$-COCl) et la réaction est effectuée de préférence dans un solvant tel que le chloroforme à une température inférieure à 30 °C, par exemple à température ambiante ou à une température voisine de 0 °C, sous atmosphère neutre, par exemple sous atmosphère d'azote.

L'hydrolyse des fonctions esters, dans la deuxième étape, peut être réalisée par dissolution du produit obtenu dans la première étape dans de l'acide chlorhydrique concentré, en excès, et chauffage à reflux pendant 5 à 15 heures environ.

L'acide phénylacétique bisphosphonique ainsi préparé est obtenu avec un excellent rendement, supérieur à 95 %.

La présente invention décrit toute composition pharmaceutique comprenant dans un support pharmaceutiquement acceptable au moins un composé de formule (I) tel que décrit ci-dessus.

Il s'agit avantageusement d'une composition pharmaceutique pour le traitement ou la prophylaxie des maladies caractérisées par un métabolisme du calcium anormal, tel que des cancers ou l'ostéoporose. Pour les cancers, il s'agit plus particulièrement des cancers du sein, de la prostate ou des leucémies.

Les tumeurs malignes solides se répartissent en carcinomes (95%) et sarcomes (5%). Le développement des carcinomes et des sarcomes est corrélé à la mise en place d'une vascularisation des tumeurs, dénommée angiogenèse. Toute inhibition de cette angiogenèse tumorale permet de faire régresser, ou pour le moins de suspendre ou de retarder le développement des tumeurs. Or, il a été trouvé de manière surprenante que les composés selon l'invention ont non seulement un effet sur les tumeurs, mais également un effet sur l'angiogenèse.

Les compositions pharmaceutiques sont utilisables notamment pour le traitement de tumeurs malignes, plus précisément le traitement de tumeurs malignes solides.

Elles sont également utilisables pour inhiber partiellement ou totalement l'angiogenèse tumorale.

Elle peuvent être en outre destinées à traiter une maladie virale ou inflammatoire hépatique.

L'invention décrit également l'utilisation d'un composé de formule (I) pour la préparation d'une composition pharmaceutique destinée à la mise en oeuvre d'une méthode de traitement ou de prophylaxie du corps humain ou animal, les composés étant représentés par la formule (I) dans laquelle $R_1$, $R_2$ et $R_3$, identiques ou différents, représentent un atome d'hydrogène, un radical alkyle, aryle, acyloxyalkyle ou hétérocycle.
A représente un radical de formule $-(CH_2)n-R_4$,
$R_4$ représente un atome d'hydrogène, un radical alkyle, un radical aryle, un hétérocycle ou un radical de formule $-NR_5R_6$,
$R_5$ et $R_6$, identiques ou différents, représentent un atome d'hydrogène ou un radical alkyle,
n est un entier de 0 à 24 inclusivement,
leurs isomères optiques et géométriques, leurs racémates, leurs sels, leurs hydrates et leurs mélanges.

Les composés décrits dans la présente invention présentent une activité spécifique, exempte d'effet secondaire et de toxicité, même dans le cas d'un traitement prolongé dans le temps. Plus particulièrement, l'acide phénylacétique bisphosphonique de formule (I) décrite ci-dessus présente une intéressante activité anti-angiogénique, anti-inflammatoire et antivirale hépatique, et une absence de toxicité. Les études et essais pharmacologiques et cliniques ont montré que les composés selon l'invention exercent des effets antitumoraux, anti-angiogéniques et pro-apoptotiques, et qu'ils sont actifs à la fois in vivo et in vitro sur la prolifération de nombreuses cellules tumorales. Ainsi, par exemple, in vitro, on a constaté que l'acide phénylacétique bisphosphonique a des effets d'inhibition de la prolifération de deux lignées cellulaires de cancer du sein, représentatives de deux types de cancer du sein (tumeur hormonosensible non métastatique et tumeur hormonoindépendante métastatique) même à faible dose, en interrompant le cycle cellulaire et en induisant une apoptose des cellules tumorales. In vivo, l'acide phénylacétique bisphosphonique bloque irréversiblement la prolifération de cellules MCF7-ras et la croissance de tumeurs mammaires dès les cinq à six premières semaines de traitement, et cet effet se prolonge sans apparition d'effet toxique, même lorsque le traitement est prolongé pendant une longue période. Les résultats in vivo et in vitro démontrent ainsi l'effet proapoptotique et antiangiogénique des composés de formule (I). Ces essais sont explicités dans les exemples ci-après.

De plus, les études effectuées ont mis en évidence une action de l'acide phénylacétique bisphosphonique sur les lésions inflammatoires hépatiques induites par des virus.

Ces résultats montrent que l'acide phénylacétique bisphosphonique et ses sels, esters partiels ou dérivés pharmaceutiquement acceptables peuvent être utilisés efficacement dans le traitement de certains cancers et plus particulièrement le cancer du sein et de la prostate, ainsi que dans le traitement des maladies inflammatoires du foie, telles que les hépatites aiguës ou chroniques, par exemple les hépatites virales ou toxiques.

L'activité antiangiogénique s'avère également utile dans le traitement des angiogénèses pathologiques, par exemple des rétinopathies du diabète, de la polyarthrite rhumatoïde et de la dégénérescence maculaire liée à l'âge.

L'invention décrit également une méthode de traitement d'une maladie caractérisée par un métabolisme du calcium anormal, tel que des cancers
ou l'ostéoporose, comprenant l'administration à un sujet, notamment humain, d'une dose efficace d'un composé ou d'une composition pharmaceutique tels que définis ci-avant.

Les compositions pharmaceutiques décrites dans la présente invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les dispersants, solubilisants, stabilisants, conservateurs, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, des huiles végétales, l'acacia, etc. Les compositions peuvent être formulées sous forme de suspension injectable, de gels, huiles, comprimés, suppositoires, poudres, gélules, capsules, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Les composés ou compositions décrits dans la présente invention peuvent être administrés de différentes manières et sous différentes formes. Ainsi, ils peuvent être injectés par voie systémique ou orale, de préférence systémique, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions, par exemple. Il est entendu que le débit et/ou la dose injectée peuvent être adaptés par l'homme du métier en fonction du patient, de la pathologie, du mode d'administration, etc. Typiquement, les composés sont administrés à des doses pouvant varier entre 0,1 $\mu$g et 500 mg/kg de poids corporel, plus généralement de 0,01 à 10 mg/kg, typiquement entre 0,1 et 10 mg/kg. En outre, des injections répétées peuvent être réalisées, le cas échéant. D'autre part, les compositions décrites dans la présente invention peuvent comprendre,

en outre, d'autres agents ou principes actifs.

D'autres aspects et avantages de la présente invention apparaîtront à la lecture des exemples qui suivent, qui doivent être considérés comme illustratifs et non limitatifs.

## Légende des Figures

Figure 1 : Schémas réactionnels du procédé de préparation de composés selon l'invention. A a la même définition que précédemment.

Figures 2 et 3 : Inhibition de la prolifération *in vitro* des cellules tumorales A 431 par BP1 (Figure 2) et BP2 (Figure 3) respectivement, après 24, 48 et 72 h de culture. Les résultats représentent le nombre de cellules ± SE (barres). *$P<0.05$ ; **$P<0.01$ par rapport aux contrôles.

Figure 4 : Inhibition de la croissance *in vivo* des tumeurs A 431 par BP1 et BP2. Les cellules A431 implantées chez la souris nude provoquent le développement de tumeurs sous cutanées. Une semaine après l'injection des cellules, les tumeurs sont traitées par BP1 et BP2 à des doses de 0.006, 0.06 et 0.6 mg/souris, 2 fois par semaine pendant 5 semaines. Les résultats représentent le volume tumoral ± SE (barres). *$P<0.05$ par rapport aux contrôles.

Figure 5 : Analyse immunohistochimique de l'angiogenèse des tumeurs A 431. Les cellules endothéliales ont été détectées avec un marqueur spécifique (GSL 1). Les résultats représentent la surface ± SE (barres) des cellules endothéliales quantifiée par analyse d'images (NIH image). *$P<0.01$ par rapport aux contrôles.

## Bibliographie générale sur le sujet.

- Adami S. and Zamberlan N. (1996)Adverse effects of bisphosphonates. A comparative review. Drug Saf. 14, 158-170.
- Boissier S., Ferrerras M., Peyruchaud O., Magnetto S., Ebetino F., Colombel, Delmas P., Delaissé JM and Clézardin P. (2000).Bisphosphonates inhibit breast and cancer prostate carcinoma cell invasion, an early event in the formation of bone metastases. Cancer Res., 60, 2949-2954.
- Boissier S., Magnetto S., Frappart L., Cuzin B., Ebetino F., Delmas P. and Clézardin P. (1997).Bisphosphonates inhibit prostate and breast carcinoma cell adhesion to unmineralized and mineralized bone extracellular matrices. Cancer Res., 57, 3890-3894.
- Diel I., Solomayer E. and Bastert G. (2000).Bisphosphonates and the prevention of metastasis: first evidences from preclinical and clinical studies. Cancer, 88, 3080-3088.
- Ezra A, Hoffman A, Breuer E, Alferiev IS, Monkkonen J, El Hanany-Rozen N, Weiss G, Stepensky D, Gati I, Cohen H, Tormalehto S, Amidon GL, Golomb G (2000).A peptide prodrug approach for improving bisphosphonate oralabsorption. J Med Chem 43, 3641-3652
- Fromigue O., Lagneaux L. and Body J. (2000).Bisphosphonates induce breast cancer cell death in vitro. J. Bone Miner. Res. 15, 2211-2221.
  Paracrine and autocrine effect of vascular endothelial growth factor : Inhibition of A431 tumor growth and angiogenesis by Carboxymethyl Benzylamide Dextran. Soumis Cell Growth and Diff.
- Hiraga T., Williams P., Mundy G. and Yoneda T. (2001).The bisphosphonate ibandronate promotes apoptosis in MDA MB 231 human breast cancer cells in bone metastases. Cancer Res., 61, 4418-4424.
- Ichinose Y., Migita K., Nakashima A., Kawakami A., Aoyagi T. and Eguchi K. (2000)Effects of bisphosphonate on the release of MMP-2 from cultured human osteoblasts. Tohoku J. Exp. Med., 192, 111-118.
- Jagdev S., Coleman R., Shipman C., Rostami H. and Croucher P. (2001).The bisphophonate, zoledronic acid, induces apoptosis of breast cancer cells : evidence for synergy with paclitaxel. Br., J. Cancer 84, 1126-1134.

- Lee M., Fong E., Singer R. and Guenette R. (2001).Bisphosphonate treatment inhibits the growth of prostate cancer cells. Cancer Res. 61, 2602-2608.

- Lin JH. (1996).Bisphosphonates: a review of their pharmacokinetic properties. Bone 18, 75-85

- Lipton A. (2000)Bisphosphonates and breast carcinoma: present and future. Cancer, 88, 3033-3037.

- Luckman SP, Hugues D., Coxon F., Graham R., Russell G. and Rogers M. (1998).Nitrogen-containing phosphonates inhibit the mevalonate pathway and prevent post-translational prenylation of GTP-binding proteins, including Ras. J. Bone Miner. Res. 13, 581-589.

- Mincey B., Moraghan T. and Perez E. (2000).Prevention and treatment of osteoporosis in women with breast cancer. Mayo Clin. Proc., 75, 821-829.

- Mondelo N., Peluffo V., Parma., Cointry G., Capozza R., Ferretti J., Piccini E. and Montuori E. (1997).Preclinical toxicology of bisphosphonates. Medicina (B Aires) 57, 93-100.

- Ogihara T., Kikuchi Y., Imai Y., Ohsaka A., Isaka M. and Oka Y. (1995).Acute lymphoblastic leukemia accompagnied by severe hyper calcemia; successful treatment with bisphosphonate. Rinsho Ketsueki 36, 29-34.

- Pade V. and Stavchansky S. (1997)Estimation of the relative contribution of the transcellular and paracellular pathway to the transport of passively absorbed drugs in the Caco-2 cell culture model. Pharm. Res. 9, 1210-1215.

- Patashnik S., Rabinovich L. and Golomb G. (1997).Preparation and evaluation of chitosan microspheres containing bisphosphonates. J. Drug Target 4, 371-380.

- Perez-Atayde A., Sallan S., Tedrow U., Connors S., Allred E. and Folkman J. (1997).Spectrum of tumor angiogenesis in the bone marrow of children with acute lymphoblastic leukemia. Am. J. Pathol. 150, 815-821.

- Ruifrok P. and Mol W. (1983)Paracellular transport of inorganic and organic ions across the rat ileum. Biochem. Pharmacol., 32, 637-640.

- Senaratne SG., Pirianov G., Mansi JL., Amett TR. and Colston KW. (2000).Bisphosphonates induce apoptosis in human breast cancer cell lines. Br. J. Cancer, 82, 1459-1468.

- Shipman C., Croucher P., Russell R., Helfrich M. and Rogers M. (1998).The bisphosphonate incadronate (YM 175) causes apoptosis of human myeloma cells in vitro by inhibiting the mevalonate pathway. Cancer Res., 58, 5294-5297.

- Shipman C., Rogers M., Apperley J.,Russell R. and Croucher P. (1997). Bisphosphonates induce apoptosis in human myeloma cell lines: a novel anti-tumor activity. Br. J. Haematol., 98, 665-672.

- Shipman C., Vanderkerken K., Rogers M., Lippitt J., Asosingh K., Hughes D., Van Camp B., Russel G.. and Croucher P. (2000a).The potent bisphosphonate ibandronate does not induce myeloma cell apoptosis in a murine model of established multiple myeloma. Br. J. Haematol. 111, 283-286.

- Takagi M., Takahiashi K., Maruyama T., Kaneko K., Obinata K., Tadokoro R., Kastumata Miura Y., Fujita H., Ishimoto K. and Yabuta K. (1998).Acute lymphoblastic leukemia accompagnied by severe hypercalcemia : successful treatment including aminohydroxypropylidene bisphosphonate (pamidronate disodium). Pediatr. Hematol. Oncol. 15, 283-286.

- Teronen O., Konttinen Y., Lindqvist C., Salo T., Ingman T., Lauhio A., Ding Y., Santavirta S., Valleala H. and Sorsa T. (1997).Inhibition of matrix metalloproteinase-1 by dichloromethylene bisphosphonate (clodronate). Calcif. Tissue Int. 61, 59-61.

- Van der Pluijm G., Vloedgraven H., Van Beek E., Van der Wee-Pals L., Lowik C. and Papapoulos S. (1996).Bisphosphonates inhibit the adhesion of breast cancer cells to bone matrices in vitro. J. Clin. Invest. 98, 698-705.

- Van der Pluijm G., Sijmons B., Vloedgraven H., Deckers M., Papapoulos S. and Lowik C. (2001).Monitoring metastatic behavior of human tumor cells in mice with species-specific polymerase chain reaction : elevated expression of angiogenesis and bone resorption stimulators by breast cancer in bone metastases. J. Bone Miner. Res. 16, 1077-1091.

- Ylitalo R., Monkkonen J. and Yla-Herttuala S. (1998).Effectes of liposome encapsulated bisphosphonates on acetylated LDL metabolism, lipid accumulation and viability of phagocyting cells. Life Sci. 62, 413-422.

- Yoneda T., Michigami T., Yi B., Williams P.J., Niewolna M. and Hiraga T. (2000). Actions of bisphosphonate on bone metastasis in animal models of breast carcinoma. Cancer 15, 2979-2988.

**EXEMPLES**

Sauf mention contraire, les pourcentages donnés sont exprimés en poids. Rdt signifie rendement.

**PREPARATION DE COMPOSES SELON L'INVENTION**

**Préparation de l'acide 1-hydroxy-1-phényléthylidène-1,1-bisphosphonique**

A un mélange de triméthylphosphite (0,01 mole) et de diméthylphosphite (0,01 mole) dans 5 ml de chloroforme, on ajoute goutte à goutte une solution de chlorure de phénylacétyle dans 5 ml de chloroforme à froid (0 °C), en maintenant le mélange réactionnel sous agitation sous atmosphère d'azote sec. Le mélange est ensuite porté à 80 °C pendant environ 8 heures.

Après refroidissement, le mélange est lavé et précipité à l'éther éthylique. Le solide blanc recueilli par filtration est l'ester de bisphosphonate (1-hydroxy-1-phényléthylidène-1,1-bisphosphonate tétraméthyl ester).
Rendement : 97 %
Point de fusion F = 120 °C
RMN $P^{31}$ ($CDCl_3$) : $\delta$ = 20,53 ppm
RMN $H^1$ (TMS, $CDCl_3$) $\delta$ = 3,8 ppm ; $\delta_{t\,(CH2)}$ = 3,4 ppm ($^3J_{HCCP}$ : 13,5 Hz) ; $\delta_{m(ph)}$= 7,3 ppm.

L'ester de bisphosphonate obtenu comme indiqué ci-dessus est re-dissout dans un large excès d'acide chlorhydrique concentré et porté à reflux pendant 15 heures. La solution aqueuse est lavée à l'éther, puis elle est évaporée sous vide sur Rotavapor. On obtient ainsi l'acide correspondant.

L'acide est purifié et reprécipité à l'éther et au mélange benzène / éther. Le précipité blanc est recueilli par filtration pour obtenir l'acide 1-hydroxy-1-phényléthylidène-1,1-bisphosphonique.
Rendement : 98 %
RMN $P^{31}$ ($D_2O$) : $\delta$ = 19,53 ppm
RMN $H^1$ ($D_2O$) : $\delta_{t(CH2)}$ = 3,4 ppm ($^3J_{HCCP}$ : 13,5 Hz) ; $\delta_{m(ph)}$= 3,4 ppm.

**Préparation du méthyl di(triméthylsilyl)phosphite**

Dans un tétracol muni d'une agitation mécanique, d'un réfrigérant, d'une ampoule à introduction, d'un thermomètre et d'une arrivée d'azote on ajoute goutte à goutte 100ml d'une solution d'ammoniaque concentrée dans 83 g de dimethylphosphonate que l'on aura si nécessaire distillé auparavant. La solution ainsi obtenue est évaporée sous pression réduite. Après des co-évaporations répétées avec de la pyridine (100ml) et du benzène (2X100ml), le solide blanc obtenu est traité par 140 ml de hexaméthyldisilazane. Le mélange ainsi obtenu est porté à reflux pendant 6 heures. La solution est ensuite distillée pour donner le composé attendu.
Rdt=58 % Eb 74-76(20 mm Hg)
$^{31}P$ {1H} RMN ($CDCl_3$): $\delta$=127.6 $^1H$ RMN ($CDCl_3$): $\delta$=0.19 (s,18H,$Me_3Si$), 3.30 (d,3H, $J_{PH}$ =8 Hz, MeO).

**Procédé de synthèse de l'acide 1-hydroxymethylene-1,1-bisphosphonique.**

Un équivalent de chlorure d'acide est placé dans un tricol pourvu d'un aimant sous atmosphère inerte (Ar). 2 équivalents de tris(trimethylsilyl)phosphite sont ajoutés à température ambiante sous agitation. La solution est ensuite laissée à température ambiante pendant quelques minutes.
L'hydrolyse est effectuée dans le méthanol à 25°C pendant 1 heure. Les fractions volatiles sont évaporées sous vide. Les produits sont purifiés par lavage à l'éther.

**Acide (1-hydroxy-1-phosphono-éthyl)-phosphonique.** Poudre blanche. Rendement = 98%.

$^{31}P$ {1H} RMN ($D_2O$): δ=19.4 $^{1}H$ RMN ($D_2O$): δ=1.48 (t, 3H, $^{3}J$= 16 Hz, $CH_3COH$).

**Acide (1-hydroxy-1-phenyl-phosphono-ethyl)-phosphonique.** Poudre blanche. Rendement=90%.

$^{31}P$ {1H} RMN ($D_2O$): δ=19.0. $^{1}H$ RMN ($D_2O$): δ=3.31 (t, 2H, $^{3}J$= 14 Hz, $CH_2COH$), 7.26-7.32 (m, 4H, $C_6H_5$), 7.38 (t, 1H, $^{3}J$= 7.5 Hz, $C_6H_5$).

**Acide (hydroxy-phenyl-phosphono-methyl)-phosphonique.** Poudre blanche. Rdt=91%.

$^{31}P$ {1H} RMN ($D_2O$): δ= 16.0. $^{1}H$ RMN ($D_2O$): δ=7.08-7.13 (m, 4H, $C_6H_5$), 7.46 (t, $^{3}J$= 5 Hz, 1H, $C_6H_5$). $^{13}C$ {1H} RMN ($D_2O$): δ=78.76 (t, $^{1}J$= 145.75 Hz, COH), 128.9 , 130.8, 131.2 , 138.6 ($C_6H_5$).

**Acide (hydroxy-*p*-nitrophényl-phosphono-methyl)-phosphonique.** Poudre blanche. Rdt= 85%.

$^{31}P$ {1H} RMN ($D_2O$): δ=15.3. $^{1}H$ RMN ($D_2O$): δ=7.89 (d, 2H, $^{3}J$= 8.5 Hz, $C_6H_4$), 8.15(d, 2H, $^{3}J$= 8.5 Hz, $C_6H_4$). $^{13}C$ {1H} RMN ($D_2O$): δ= 75.28 (t, $^{1}J$= 149 Hz, COH), 124.67, 128.15 ;146.13 , 148.35 ($C_6H_4$).

**Mode opératoire général des produits du type A-C(OH)PO$(OH)_2$PO$(OMe)_2$**

Dans un tétracol muni d'une agitation mécanique, d'un réfrigérant, d'une ampoule à introduction, d'un thermomètre et d'une arrivée d'azote on ajoute goutte à goutte 1 équivalent de trimethylphosphite dans 1 équivalent de chlorure d'acide à 0 °C. Après deux heures de réaction à température ambiante, l'α-cétophosphonate de diméthyle est complètement formée (l'évolution de la réaction est suivie en RMN $^{31}P$). On ajoute ensuite goutte à goutte 1 équivalent de tris (triméthylsilyl)phosphite. La solution est agitée ensuite pendant 15 minutes. Le méthanol est ensuite ajouté et la solution agitée pendant deux heures à température ambiante. Le solvant et les fractions volatiles sont ensuite évaporés.
Le produit obtenu se présente sous forme de poudre blanche. Il est ensuite purifié par des lavages successifs à l'éther.

**Acide [(Diméthoxy-phosphoryl)-hydroxy-phényl-méthyl]-phosphonique** Poudre blanche. Rdt=91%

$^{31}P$ {1H} RMN ($D_2O$): δ=23.58 (d, $^{2}J$= 27 Hz) ; 12.92 (d, $^{2}J$= 27 Hz) . $^{1}H$ RMN ($D_2O$): δ=7.73 (d, 2H, $^{3}J$= 6.0 Hz, $C_6H_5$) ; 7.46-7.43(m, 3H" $C_6H_5$) ; 3.79(d, 3H, $^{3}J$= 10.0 Hz, $OCH_3$) ; 3.63 (d, 3H, $^{3}J$= 10.0 Hz, $OCH_3$).

**Acide [(Diméthoxy-phosphoryl)-hydroxy-éthyl]-phosphonique** Poudre blanche. Rdt=91%

$^{31}P$ {1H} RMN ($D_2O$): δ=29.1 (d, $^{2}J$= 27 Hz) ; 19.90 (d, $^{2}J$= 27 Hz). $^{1}H$ RMN ($D_2O$): δ=7.73 (d, 2H, $^{3}J$= 6.0 Hz, $C_6H_5$) ; 7.46-7.43(m, 3H" $C_6H_5$) ; 3.79(d, 3H, $^{3}J$= 10.0 Hz, $OCH_3$) ; 3.63 (d, 3H, $^{3}J$= 10.0 Hz, $OCH_3$).

**Mode opératoire général des produits du type A-C(OH)PO(OH)OMePO(OH)(OMe)**

Dans un tétracol muni d'une agitation mécanique, d'un réfrigérant, d'une ampoule à introduction, d'un thermomètre et d'une arrivée d'azote on ajoute goutte à goutte 2 équivalents de methyl bis(trimethylsilyl)phosphite dans 1 équivalent de chlorure. Après 10 minutes de réaction à température ambiante, la solution est méthanolysée pendant deux heures. Le solvant et les fractions volatiles sont ensuite évaporés. Le produit obtenu se présente sous forme de poudre blanche. Il est ensuite purifié par des lavages successifs à l'éther.

**Monométhyl ester de l'acide [Hydroxy-(hydroxy-méthoxy-phosphoryl)-phényl-méthyl] phosphonique.**

Poudre blanche. Rdt =90 %
$^{31}P$ {1H} RMN ($D_2O$): δ=18,50 . $^{1}H$ RMN ($D_2O$): δ=7.43-7.41 (m, 2H, $C_6H_5$) ; 7.15-7.05 (m, 3H, $C_6H_5$) ; 3.79(d, 6H, $^{3}J$= 6.0 Hz, $OCH_3$)

**Monométhyl ester de l'acide [([Hydroxy-(hydroxy-méthoxy-phosphoryl)-éthyl] phosphonique**

Poudre blanche. Rdt=90%
$^{31}P$ {1H} RMN ($D_2O$): δ=23.9. $^{1}H$ RMN ($D_2O$): 2.96(d, 6H, $^{3}J$= 6.0 Hz, $OCH_3$) ; 1.07-0.98 (m, 3H, $CH_3$).

**Monométhyl ester de l'acide [Hydroxy-(hydroxy-méthoxy-phosphoryl)-2-phényl-éthyl] phosphonique.**

Poudre blanche. Rdt=90%
$^{31}P$ {1H} RMN ($D_2O$): δ=20.79 . $^{1}H$ RMN ($D_2O$): δ=7.26-7.11 (m, 5H, $C_6H_5$) 3.79(d, 6H, $^{3}J$= 6.0 Hz, $OCH_3$) ; 3.15 (t, 2H,$^{3}J$= 10.0 Hz, $C_6H_5$-$CH_2$)

**Mode opératoire général des produits du type A-C(OH)PO(OH)(OMe)PO(OMe)$_2$**

Dans un tétracol muni d'une agitation mécanique, d'un réfrigérant, d'une ampoule à introduction, d'un thermomètre et d'une arrivée d'azote on ajoute goutte à goutte 1 équivalent de trimethylphosphite dans 1 équivalent de chlorure d'acide à 0 °C. Après deux heures de réaction à température ambiante, l'α-cétophosphonate de diméthyle est complètement formée (l'évolution de la réaction est suivie en RMN $^{31}P$) On ajoute ensuite goutte à goutte 1 équivalent de méthyl bis(triméthylsilyl)phosphite. La solution est agitée ensuite pendant 15 minutes. Le méthanol est ensuite ajouté et la solution est agitée pendant deux heures à température ambiante. Le solvant et les fractions volatiles sont ensuite évaporés.
Le produit obtenu se présente sous forme d'huile incolore. Il est ensuite purifié par des lavages successifs à l'éther.

**Diméthyl ester de l'acide [Hydroxy-(hydroxy-méthoxy-phosphoryl)-phényl-méthyl]-phosphonique**

Poudre blanche. Rdt= 90 %.
$^{31}P$ {1H} RMN ($CDCl_3$): δ=16.74 (d, $^{2}J$= 47 Hz) ; 14.52 (d, $^{2}J$= 47 Hz) . $^{1}H$ RMN ($D_2O$): δ=8.60-8.40 (s, 2H, OH) ; 7.49 (d, 2H, $^{3}J$= 8.0 Hz, $C_6H_5$) ; 7.04-6.97(m, 3H" $C_6H_5$); 3.47(d, 3H, $^{3}J$= 10.0 Hz, $OCH_3$) ; 3.34 (d, 3H, $^{3}J$= 10.0 Hz, $OCH_3$) ; 3.23 (d, 3H, $^{3}J$= 10.0 Hz, $OCH_3$).

**Diméthyl ester de l'acide [Hydroxy-(hydroxy-methoxy-phosphoryl)-ethyl]-phosphonique**

$^{31}P$ {1H} RMN ($CDCl_3$): δ=20.46 (d, $^{2}J$= 48 Hz) ; 18.67 (d, $^{2}J$= 48 Hz). $^{1}H$ RMN ($D_2O$): δ=8.98 (s, 2H, OH) 3.92 (d, 3H, $^{3}J$= 10.0 Hz, $OCH_3$) ; 3.88 (d, 3H, $^{3}J$= 10.0 Hz, $OCH_3$) ; 3.86 (d, 3H, $^{3}J$= 10.0 Hz, $OCH_3$) ; 1.67 (t,3H, $^{3}J_{H-P}$=15.5 Hz).

**ACTIVITES ANTI-PROLIFERATIVES ET ANTI-INVASIVES DE COMPOSES SELON L'INVENTION**

Deux bisphosphonates dénommés BP1 et BP2 dont les formules figurent ci-dessous ont été testés sur des modèles cellulaires et animaux. BP1 n'étant pas partiellement estérifié correspond à un exemple comparatif.

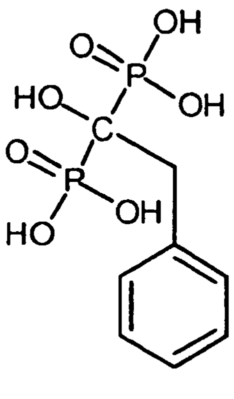
BP1

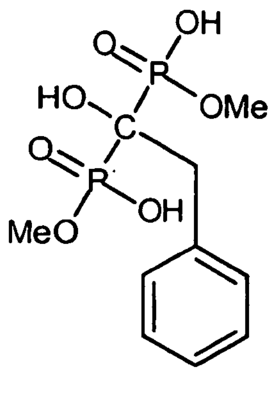
BP2

**Tests *in vitro***

Matériel et méthodes :

Les bisphosphonates BP1 et BP2 ont été testés sur différentes lignées cellulaires :

- FRCjun MRA, lignée de fibrosarcome murin après transfection de cellules FR3T3 par l'oncogène *jun*-4,

- Cellules A 431 de tumeur épidermoïde humaine, ces cellules présentent une boucle autocrine au VEGF (vascular endothelial growth factor) et entraînent une fois implantées chez des souris immunodéprimées un développement rapide de tumeurs fortement angiogéniques,
- Cellules MDA MB 435 de tumeur mammaire humaine,
- Cellules HUV-EC-C, cellules endothéliales de cordon ombilical humain (HUVEC) transformées,
- Cellules BBC, cellules endothéliales de capillaires cérébraux bovins.

La prolifération cellulaire en présence de différentes doses de BP1 ou de BP2 à été mesurée par un test MTT après 24, 48 et 72 h de culture.

Résultats *in vitro* :

Les bisphosphonates (0,1-2 mM) inhibent la prolifération des cellules FRCjunMRA, MDA MB 435 et A 431 de manière temps et dose dépendants (Fig. 2 et 3). A titre d'exemple BP2 (2 mM) inhibe la prolifération des 3 lignées cellulaires respectivement de 40%, 60% et 30% après 48 h de culture. Un temps de traitement de 72 h par BP2 (2 mM) entraîne une inhibition de 80% de la prolifération des cellules A 431 ($P<0.05$) (Fig. 3).

Un test d'exclusion au bleu trypan montre que BP1 et BP2 n'induisent pas de toxicité cellulaire, même à des concentrations supérieures à 5 mM.

Par ailleurs, les bisphosphonates (0,1-2 mM) inhibent également la prolifération des cellules endothéliales HUV EC C et BBC de manière temps et dose dépendants.

Ces résultats ont été confirmés par un test de morphogenèse vasculaire *in vitro*, dans lequel classiquement des cellules endothéliales sont déposées sur une matrice extracellulaire (Matrigel) ; dans les conditions témoins, les cellules endothéliales s'assemblent pour former des structures en pseudo-capillaires. Le traitement, au moment de l'ensemencement, des cellules endothéliales par BP1 et BP2 prévient la formation des structures tubulaires.

Des études par zymographie ont également montré que les cellules A431 secrètent de la métalloprotéinase 2 qui à une action pro-métastatique. Le traitement par BP1 et BP2 inhibe l'activité enzymatique de la métalloprotéinase 2, les BP présentent ainsi, en plus de leur activité anti-angiogénique, une activité anti-métastatique.

**Tests de toxicité**

Des souris immunodéprimées, non tumorées, ont été traitées par BP1 et BP2 à différentes concentrations s'échelonnant de 0,06 à 6mg par injection et par souris. Les souris recevaient une injection par jour pendant une semaine. Le poids des animaux était mesuré avant, pendant et après le traitement.

Les doses de BP1 inférieures à 6 mg/injection et toutes les doses de BP2 testées (0,06 - 6 mg/injection) n'ont pas montré de signes de toxicité tels perte de poids, diarrhée, infection ou anémie chez les animaux traités.

**Tests *in vivo***

Matériel et méthodes :

Des cellules A 431 ont été injectées en sous cutanée chez des souris immunodéprimées. Les souris développaient des tumeurs en une semaine. Les souris porteuses de tumeurs étaient placées arbitrairement en groupe témoin, et des groupes traités par BP1 et BP2 à différentes concentrations.

Les souris recevaient en sous cutanée à proximité de la tumeur deux fois par semaine, 0,1 ml de PBS seul pour les témoins (n=6), ou du PBS contenant soit BP1 soit BP2 aux doses suivantes : 0,006 (n=6) ; 0,06 (n=6) ou 0,6 mg/injection (n=6). Les tumeurs étaient mesurées toutes les semaines et leur volume V déterminé par la formule :

$$V = (4/3)\ \pi R1^2 R2$$

où R1 est le rayon 1 et R2 le rayon 2, avec R1<R2.

Après sacrifice des animaux et prélèvements des tumeurs, ces dernières étaient pesées fixées, incluses en paraffine puis coupées. Les cellules endothéliales des vaisseaux tumoraux mis en place durant l'angiogenèse tumorale étaient visualisées par des techniques immunohistochimiques (GSL 1). L'angiogenèse tumorale a été déterminée par analyse d'images à l'aide d'un programme NIH image.

Résultats *in vivo*

Le traitement avec les bisphosphonates inhibent la croissance des tumeurs A 431 et est maximale même aux doses les plus faibles (Fig. 4). Après 5 semaines de traitement, BP1 et BP2 (0,6mg/injection/souris) inhibent la croissance des tumeurs A 431 respectivement de 40% et 56% ($P<0.05$).

Au contraire des résultats obtenus *in vitro,* les résultats *in vivo* montrent que BP2 est plus efficace que BP1.

Par ailleurs, l'inhibition de la croissance des tumeurs A 431 chez les animaux traités est corrélée à une inhibition de l'angiogenèse intratumorale (Fig. 5). Cette inhibition, démontrée par une baisse significative de la surface occupée par les cellules endothéliales par unité de surface, est notée dès les plus faibles valeurs de BP1 et BP2.

De plus, nous avons étudié l'effet des bisphosphonates par un test *in vivo* d'angiogenèse en Matrigel. Classiquement du VEGF exogène (facteur pro-angiogénique), déposé dans le Matrigel injecté en sous cutané chez la souris, provoque l'invasion de cette matrice artificielle par les cellules endothéliales et la formation de structures vasculaires. L'étude microscopique du Matrigel retiré après 10 jours des animaux traités montre que BP1 et BP2 inhibent la migration des cellules endothéliales dans la matrice empêchant ainsi la formation de structures capillaires.

A notre connaissance, les bisphosphonates synthétisés sont les premiers à présenter une action anti tumorale *in vivo* sur la tumeur primaire, leurs effets étant corrélés à une baisse de l'angiogenèse.

**Etude *in vitro* du phénylacétate bisphosphonate de sodium**

L'étude de l'effet du phénylacétate bisphosphonate de sodium sur la croissance et la viabilité cellulaire a été effectuée de la manière suivante.

Des cellules MCF7-ras sont ensemencées à raison de 20 000 cellules par puits, dans des plaques de 24 puits. Après 24 heures d'incubation, le milieu (DMEM avec 10 % de sérum de veau foetal) est remplacé par un même milieu additionné de différentes concentrations de phénylacétate bisphosphonate (2 mM, 4 mM, 6 mM, 8 mM et 10 mM). Les cellules sont incubées pendant 1 à 4 jours à 37 °C. La viabilité cellulaire est vérifiée par le test du bleu trypan.

On constate alors que le phénylacétate biphosphonate est cytostatique pendant les trois premiers jours de culture à des concentrations inférieures à 6 mM. Aux concentrations plus élevées (8 et 10 mM) une légère toxicité (10 %) est observée, qui devient importante (50-60 %) au quatrième jour de culture.

Afin de vérifier la réversibilité de l'effet du phénylacétate bisphosphonate, des cellules MCF7-ras ont été traitées avec des concentrations croissantes de phénylacétate bisphosphonate pendant 10 jours, en changeant le milieu tous les deux jours. Puis les cellules sont réparties en deux lots. Au premier lot on ajoute le milieu de culture DMEM complémenté avec 10 % de sérum de veau foetal, et au deuxième lot on ajoute le même milieu avec 10 mM de phénylacétate bisphosphonate, en changeant le milieu tous les jours. Au 15$^{ème}$ jour on ajoute de la thimidine tritiée pendant 4 heures et la radioactivité de la suspension est déterminée au moyen d'un compteur à scintillation liquide bêta (Beckman).

On observe alors une inhibition de la prolifération cellulaire partiellement réversible à la dose de 2 mM et irréversible au-delà de 4 mM.

**Activité proapoptotique du phénylacétate bisphosphonate de sodium**

Afin de déterminer la nature de la toxicité du phénylacétate bisphosphonate de sodium, des cellules MCF7 et MCF7-ras sont ensemencées, puis lavées au bout de 24 heures et ensemencées pendant 3 heures dans du milieu DMEM additionné de 10 % de sérum de veau foetal. Un lot est additionné de phénylacétate bisphosphonate (10 mM) tandis que l'autre lot n'en contient pas. L'apoptose est déterminée par le test de l'Annexine V conjuguée à l'anticorps FITC. L'iodure de propidium est utilisé pour distinguer l'apoptose précoce (marquage positif pour l'Annexine V et négatif pour l'iodure de propidium) de l'apoptose tardive (marquage positif pour l'Annexine V et l'iodure de propidium).

On constate qu'au bout de 4 heures de traitement avec le phénylacétate bisphosphonate, le pourcentage de cellules en apoptose précoce est presque identique pour les deux types cellulaires, tandis que celui des cellules en apoptose tardive est plus importante pour les cellules MCF7-ras (22 % et 53 % respectivement).

L'apoptose des cellules est confirmée par la méthode de la dégradation de l'ADN. A cet effet les cellules MCF7 et MCF7-ras sont ensemencées à raison de $5 \times 10^5$ cellules dans des flacons T25. Après 24 heures, les cellules sont lavées puis ensemencées dans du milieu de DMEM additionné de 10 % de sérum de veau foetal, avec phénylacétate bisphosphonate (10 mM) pour un lot et sans phénylacétate bisphosphonate pour l'autre lot.

Après 96 heures, l'extrait cellulaire contenant l'ADN fragmenté est incubé avec 0,5 mg/ml de RNase A à 37 °C pendant une heure puis avec 0,5 mg/ml de protéinase K pendant une heure à 37 °C. Après l'incubation, l'ADN fragmenté est précipité par l'isopropanol puis dissous dans 10 mM de Tris-HCl (pH 8) 1 ml d'EDTA, 5 % de glycérol et 0,05 % de bleu de bromophénol. L'ADN fragmenté séparé par électrophorèse sur gel d'agarose à 1 % est marqué par le bromure d'éthidium et photographié en UV.

On observe alors une nette dégradation de l'ADN des cellules MCF7 et MCF7-ras dans le cas du lot contenant

10 mM de phénylacétate bisphosphonate.

Ces résultats montrent que l'acide phénylacétate bisphosphonate inhibe la prolifération et induit l'apoptose des cellules MCF7 et MCF7-ras.

**Etude *in vivo* du phénylacétate bisphosphonate de sodium**

L'effet antitumoral du phénylacétate bisphosphonate de sodium est vérifié chez la souris.

L'inoculation de 4 x $10^6$ cellules MCF7-ras chez des souris nude femelles athymiques est effectuée par voie sous-cutanée dans un volume de 0,1 ml de DMEM et induit 70 % de prise de tumeurs après trois semaines.

Le traitement par le phénylacétate bisphosphonate a commencé quand les tumeurs avaient un volume moyen de 550 $mm^3$. Les animaux sont répartis en deux lots, l'un avec phénylacétate bisphosphonate, l'autre sans. Le phénylacétate bisphosphonate est injecté par voie sous-cutanée et péritumorale, deux fois par semaine, pendant 5 semaines, en utilisant des doses de 80 mg/kg (5 cas) et 160 mg/kg (6 cas).

En fin de traitement, on observe une inhibition de la croissance des tumeurs qui est irréversible et dépendante de la dose. Après l'arrêt du traitement, le croissance des tumeurs est bloquée pendant au moins trois semaines pour les souris traitées avec une dose de 160 mg/kg tandis que la croissance reprend dans le cas des souris traitées avec une dose de 80 mg/kg.

L'examen immunohistologique des tumeurs montre que la diminution de la prolifération est liée à l'induction de l'apoptose, à une forte diminution de l'angiogénèse de la tumeur et à l'induction de la fibrose dans la tumeur.

**Effet pro-apoptotique**

De plus, l'effet proapoptotique du phénylacétate bisphosphonate est confirmé par la technique du marquage par anticorps. Le marquage de cellules apoptotiques est effectué par l'anticorps M30 qui reconnaît le site spécifique des capsases sur les filaments de cytokératine 18 produits par les cellules épithéliales. Ces filaments s'agrègent rapidement dans les cellules en apoptose précoce, par hyperphosphorylation des cytokératines. Ce marquage cytoplasmique permet d'observer des structures granulaires à l'intérieur du cytoplasme, qui correspondent aux cellules en apoptose tardive.

Ces structures granulaires sont observées dans les tumeurs traitées avec une forte dose (160 mg/kg) de phénylacétate bisphosphonate. On constate aussi une augmentation du marquage avec l'anticorps anticytokératine 18 en fonction de la concentration. L'apoptose précoce peut ainsi être distinguée de l'apoptose tardive par la présence des structures granulaires dans le cytoplasme.

Ceci confirme que le traitement des tumeurs par le phénylacétate bisphosphonate à une dose de 80 mg/kg provoque une apoptose alors que le traitement à la dose de 160 mg/kg provoque une aponécrose, phase intermédiaire entre l'apoptose et la nécrose.

**Effet anti-angiogénique**

L'effet anti-angiogénique a été vérifié par la technique de la lectine GSL1.

En utilisant la lectine GSL1 on détermine l'angiogénèse, identifiée par le marquage des cellules endothéliales en rouge avec l'anticorps anti-GSL1. On constate que cette angiogénèse est inhibée par le traitement des tumeurs par le phénylacétate bisphosphonate. Cet effet est partiel à la dose de 80 mg/kg et total à 160 mg/kg.

Ces essais démontrent que l'acide phénylacétate bisphosphonate présente un effet antiangiogénique très important, ainsi qu'un effet antitumoral et proapoptotique. Ces résultats confirment que l'acide phénylacétate bisphosphonate constitue un principe actif potentiel de médicament pour le traitement des tumeurs cancéreuses, en particulier le cancer du sein.

**Effet sur les cellules inflammatoires hépatiques**

L'étude est effectuée sur des souris nude développant une hépatite aiguë. Les souris sont réparties en deux lots, l'un étant traité par le phénylacétate bisphosphonate de sodium, l'autre pas. Les souris traitées reçoivent le phénylacétate bisphosphonate à raison de 80 mg/kg ou 160 mg/kg par voie sous-cutanée, deux fois par semaine pendant 5 semaines. Puis les animaux sont sacrifiés et le foie est prélevé et analysé histologiquement après HES (méthode de la coloration par Hematoxilline Eosine, ou Hemalun).

L'examen au microscope optique montre une atteinte très importante des foies de souris non traitées par rapport au foie des souris traitées. On observe en particulier des zones nécrotiques dues à une hépatite chronique, ainsi qu'une accumulation de liquides dans les hépatocytes (stéatose) assoicée à une inflammation des cellules lymphocytaires. Cette atteinte hépatique provoque la "nécrose des tissus hépatiques, se traduisant par la formation d'une zone

fibreuse.

Au contraire, chez les souris traitées par le phénylacétate bisphosphonate, dans 4 cas sur 5, on n'observe aucun signe de toxicité hépatique.

## Revendications

**1.** Procédé de préparation de dérivés de bisphosphonate de formule (I) suivante:

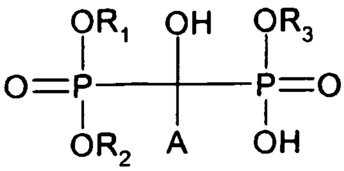

(I),

avec $R_1$, $R_2$ et $R_3$, identiques ou différents, représentent un atome d'hydrogène, un radical alkyle, aryle, acyloxyalkyle, ou hétérocycle, dans laquelle $R_1$ et $R_2$ sont différents de l'atome d'hydrogène,
A représente un radical de formule $-(CH_2)n-R_4$,
$R_4$ représente un atome d'hydrogène, un radical alkyle, un radical aryle, un hétérocycle ou un radical de formule $-NR_5R_6$,
$R_5$ et $R_6$, identiques ou différents, représentent un atome d'hydrogène ou un radical alkyle,
n est un entier de 0 à 24 inclusivement,
**caractérisé en ce qu'**il comprend les étapes suivantes :

- la mise en contact d'au moins un halogénure d'acide de formule (II) : ACOX avec au moins un phosphite de formule (V) : P(OR1)(OR2)(OR), avec R1, R2 et R, identiques ou différents, représentent un radical alkyle, aryle, acyloxyalkyle, ou hétérocycle, pour former un $\alpha$-cétophosphonate de formule (III),
- la mise en contact du $\alpha$-cétophosphonate obtenu précédemment de formule (III) :

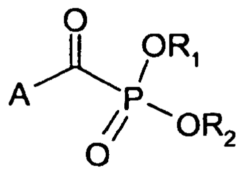

avec au moins un phosphite silylé de formule (IV) :

$$P[OSialk_3]_x [OR_3]_{3-x} \qquad (IV)$$

formule (IV) dans laquelle $R_3$ représente un radical alkyle, aryle, acyloxyalkyle, ou hétérocycle,
A étant tel que défini ci-dessus,
X représente un atome d'halogène, de préférence le chlore,
alk représente un radical alkyle de C1-6,
x représente 2 ou 3,

- une hydrolyse des composés obtenus à l'étape précédente, lesdits radicaux alkyle, aryle, hétérocycle et acyloxyalkyle précédemment cités sont éventuellement substitués par au moins un groupement choisi parmi un radical aryle, un radical hétérocycle, un radical hétérocycloalkyle, un radical alkyle, un radical alkényle, un radical alkynyle, un radical alkylthio, un atome d'halogène, de préférence Cl, F, Br, un radical hydroxyle, un groupement $NO_2$, un radical alkoxy, un groupe ester (-COOR), un groupe aminé -NRR''', une fonction acide, un groupement amide (-CONHR ou -NHCOR), où R et R''' représentent, indépendamment l'un de l'autre, un atome d'hydrogène, un radical alkyle, aryle, hétéroaryle ou acyloxyalkyle.

**2.** Procédé selon la revendication précédente, **caractérisé en ce que** le phosphite silylé est un composé de formule (IV) dans laquelle x vaut 2.

**3.** Procédé selon la revendication 1, **caractérisé en ce que** le phosphite silylé est un composé de formule (IV) dans laquelle x vaut 3.

**4.** Procédé de préparation de dérivés de bisphosphonate de formule (I) suivante:

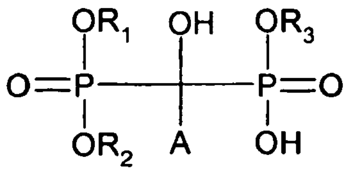

(I),

avec $R_1$ et $R_3$ représentent un radical méthyle et $R_2$ représente un atome d'hydrogène, A étant tel que defini dans la revendication 1. **caractérisé en ce qu'**il comprend les étapes suivantes :

- la mise en contact un équivalent molaire d'un chlorure d'acide de formule (II) : ACOCl, avec A étant tel que défini ci-dessus, avec deux équivalents-molaires d'un phosphite silylé de formule $P[OSiMe_3]_2[OMe]$,
- une hydrolyse des composés obtenus à l'étape précédente.

**5.** Procédé de préparation de dérivés de bisphosphonate de formule (I) suivante:

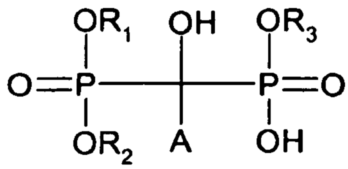

(I),

avec $R_1$ représente un radical alkyle, aryle, acyloxyalkyle, ou hétérocycle, et $R_2$ et $R_3$ représentent un atome d'hydrogène, A et les éventuels substituants des radicaux précédemment cités étant tels que définis dans la revendication 1, **caractérisé en ce qu'**il comprend les étapes suivantes :

- la mise en contact au moins un halogénure d'acide de formule (II) : ACOX, tel que défini à la revendication 1, avec au moins un phosphite silylé de formule (IV) tel que défini dans la revendication 1 dans laquelle x vaut 2, puis la mise en contact du produit ainsi obtenu avec au moins un phosphite silylé de formule (IV) tel que défini dans la revendication 1 dans laquelle x vaut 3,
- une hydrolyse des composés obtenus à l'étape précédente.

**6.** Procédé selon l'une quelconque des revendications précédentes, **caractérisé en ce que** A représente un radical de formule $-(CH_2)n-R_4$, dans laquelle $R_4$ représente un radical alkyle C1-C6, un radical hétérocycle, un radical phényle, ou un radical de formule $-NR_5R_6$, dans laquelle $R_5$ et $R_6$, identiques ou différents, représentent un atome d'hydrogène ou un radical alkyle de C1-6, dans laquelle avantageusement n est entier de 1 à 6 inclusivement, de préférence de 1 à 3.

**7.** Procédé selon l'une quelconque des revendications précédentes, **caractérisé en ce que** A représente un radical choisi parmi :

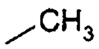 ; 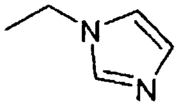 ; 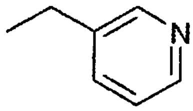 ; 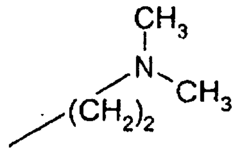 ;

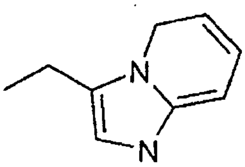 ; 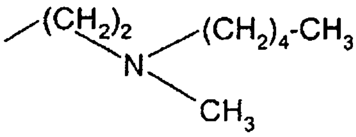 ; 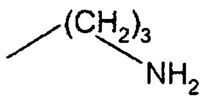 ;

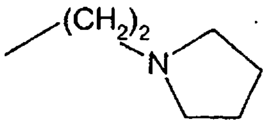 ; 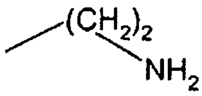 ; et 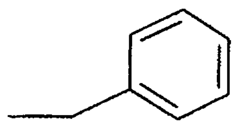 .

## Claims

**1.** Method of preparation of bisphosphonate derivatives represented by the following formula (I):

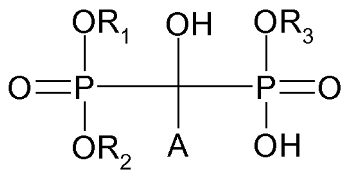

(I)

wherein $R_1$, $R_2$ and $R_3$, which are the same or different, represent a hydrogen atom, an alkyl, aryl, acyloxyalkyl or heterocycle group, wherein
$R_1$ and $R_2$ are different from a hydrogen atom,
A represents a group with the formula $-(CH_2)n-R_4$,
$R_4$ represents a hydrogen atom, an alkyl group, an aryl group, a heterocycle or a group with the formula $-NR_5R_6$,
$R_5$ and $R_6$, which are the same or different, represent a hydrogen atom or an alkyl group,
n is a whole number from 0 to 24 inclusive,
**characterized in that** it comprises the following steps :

- contacting at least one acid halide represented by formula (II): ACOX with at least one phosphite represented by formula (V) : P(OR1)(OR2)(OR), wherein $R_1$, $R_2$ and R, which are the same or different, represent an alkyl, aryl, acyloxyalkyl, or heterocycle group, to form an $\alpha$-ketophosphonate represented by formula (III),
- contacting the previously obtained $\alpha$-ketophosphonate represented by formula (III) :

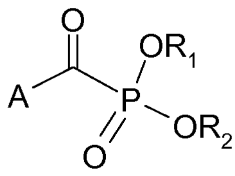

with at least one silyl phosphite represented by formula (IV) :

$P[OSialk_3]_x[OR_3]_{3-x}$ (IV)

formula (IV) wherein $R_3$ represents an alkyl, aryl, acyloxyalkyl, or heterocycle group,
A being as defined above,
X represents a halogen atom, preferably chlorine,
alk is a C1-6 alkyl group,
x is equal to 2 or 3,
- hydrolysis of the compounds obtained in the previous step,

the previously cited alkyl, aryl, acyloxyalkyl or heterocycle group are optionally substituted by at least one group chosen from among an aryl group, a heterocycle group, a heterocycloalkyl group, an alkyl group, an alkenyl group, an alkynyl group, an alkylthio group, a halogen atom, preferably Cl, F, Br, a hydroxyl group, an $NO_2$ group, an alkoxy group, an ester group (-COOR), an amino group -NRR''', an acid moiety, an amide group (-CONHR or -NHCOR), wherein R and R''' represent, independently of each other, a hydrogen atom, an alkyl, aryl, heteroaryl or acyloxyalkyl group.

2. Method according to claim 1, wherein the silyl phosphite is a compound represented by formula (IV) in which x is equal to 2.

3. Method according to claim 1, wherein the silyl phosphite is a compound represented by formula (IV) in which x is equal to 3.

4. Method of preparation of bisphosphonate derivatives represented by the following formula (I):

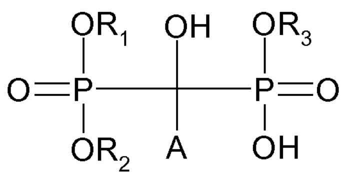

wherein $R_1$ and $R_3$ represent a methyl group and $R_2$ is a hydrogen atom,
A being as defined in claim 1,
**characterized in that** it comprises the following steps :

- contacting one molar equivalent of an acid chloride represented by formula (II): ACOCl, with A being as defined above, with two molar equivalent of silyl phosphite of formula $P[OSiMe_3]_2[OMe]$,
- hydrolysis of the compounds obtained in the previous step.

5. Method of preparation of bisphosphonate derivatives represented by the following formula (I):

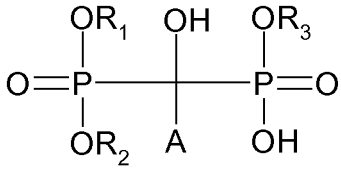

wherein $R_1$ represents an alkyl, aryl, acyloxyalkyl, or heterocycle group, and $R_2$ and $R_3$ represent a hydrogen atom, A and the optional substituents of the previously cited groups being as defined in claim 1,
**characterized in that** it comprises the following steps:

- contacting at least one acid chloride represented by formula (II): ACOCl, as defined in claim 1, with at least one silyl phosphite of formula (IV) as defined in claim 1 wherein x is 2, then contacting the obtained product with at least one silyl phosphite of formula (IV) as defined in claim 1 wherein x is 3,
- hydrolysis of the compounds obtained in the previous step.

**6.** Method according to one of the preceding claims, wherein A represents a group with the formula $-(CH_2)n-R_4$, in which $R_4$ is a C1-C6 alkyl group, a heterocycle group, a phenyl group, or a group with the formula $-NR_5R_6$, wherein $R_5$ and $R_6$, which are the same or different, represent a hydrogen atom or a C1-6 alkyl group, in which n is advantageously a whole number from 1 to 6 inclusive, preferably from 1 to 3.

**7.** Method according to one of the preceding claims, wherein A is a group chosen from:

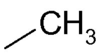 ; 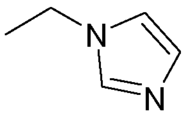 ; 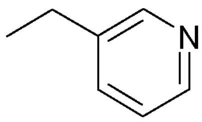 ; 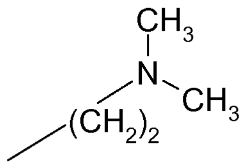 ;

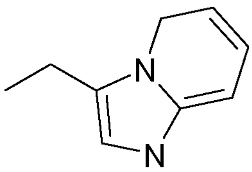 ; 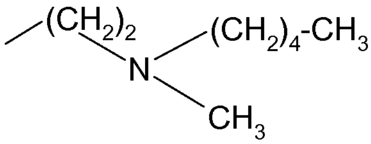 ; 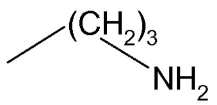 ;

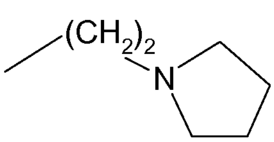 ; 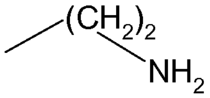 ; or 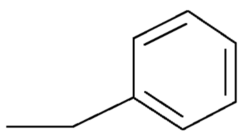

**Patentansprüche**

**1.** Verfahren zur Herstellung von Derivaten von Bisphosphonat der folgenden Formel (I):

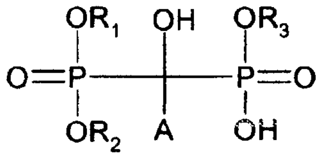

(I),

wo $R_1$, $R_2$ und $R_3$ identisch oder verschieden sind und für ein Wasserstoffatom, einen Alkyl-, Aryl-, Acyloxyalkyl- oder Heterocyclusrest stehen, in der $R_1$ und $R_2$ sich vom Wasserstoffatom unterscheiden,
A für einen Rest der Formel $-(CH_2)_n-R_4$ steht,
$R_4$ für ein Wasserstoffatom, einen Alkylrest, einen Arylrest, einen Heterocyclus oder einen Rest der Formel $-NR_5R_6$ steht,
$R_5$ und $R_6$ identisch oder verschieden sind und für ein Wasserstoffatom oder einen Alkylrest stehen,
n eine ganze Zahl von 0 bis einschließlich 24 ist,
**dadurch gekennzeichnet, dass** es die folgenden Schritte umfasst:

- Inkontaktbringen wenigstens eines Säurehalogenids der Formel (II): ACOX mit wenigstens einem Phosphit der Formel (V): P(OR1)(OR2)(OR), wo R1, R2 und R identisch oder verschieden sind und für einen Alkyl-, Aryl-, Acyloxyalkyl- oder Heterocyclusrest stehen, um ein α-Ketophosphonat der Formel (III) zu bilden,
- Inkontaktbringen des zuvor erhaltenen α-Ketophosphonats der Formel (III):

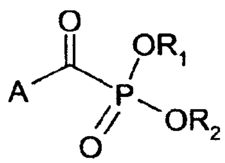

mit wenigstens einem silylierten Phosphit der Formel (IV):

$$P[OSiAlk_3]_x[OR_3]_{3-x} \qquad (IV)$$

in der $R_3$ für einen Alkyl-, Aryl-, Acyloxyalkyl- oder Heterocyclusrest steht,
A wie oben definiert ist,
X für ein Halogenatom, vorzugsweise Chlor, steht,
Alk für einen Alkylrest von C1-6 steht,
x für 2 oder 3 steht,
- eine Hydrolyse der im vorhergehenden Schritt erhaltenen Verbindungen, besagte Alkyl-, Aryl-, Heterocyclus- und Acyloxyalkylreste, die zuvor genannt werden, eventuell mit wenigstens einer Gruppe substituiert sind, die aus einem Arylrest, einem Heterocyclusrest, einem Heterocycloalkylrest, einem Alkylrest, einem Alkenylrest, einem Alkinylrest, einem Alkylthiorest, einem Halogenatom, vorzugsweise Cl, F, Br, einem Hydroxylrest, einer $NO_2$-Gruppe, einem Alkoxyrest, einer Estergruppe (-COOR), einer Aminogruppe -NRR''', einer Säurefunktion, einer Amidgruppe (-CONHR oder -NHCOR), wo R und R''' unabhängig voneinander für ein Wasserstoffatom, einen Alkyl-, Aryl-, Heteroaryl- oder Acyloxyalkylrest stehen, ausgewählt ist.

2. Verfahren gemäß dem vorhergehenden Anspruch, **dadurch gekennzeichnet, dass** das silylierte Phosphit eine Verbindung der Formel (IV) ist, in der x den Wert 2 besitzt.

3. Verfahren gemäß Anspruch 1, **dadurch gekennzeichnet, dass** das silylierte Phosphit eine Verbindung der Formel (IV) ist, in der x den Wert 3 besitzt.

4. Verfahren zur Herstellung von Derivaten von Bisphosphonat der folgenden Formel (I):

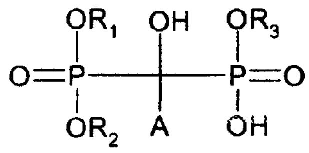

(I),

wo $R_1$ und $R_3$ für einen Methylrest stehen und $R_2$ für ein Wasserstoffatom steht,
A wie in Anspruch 1 definiert ist,
**dadurch gekennzeichnet, dass** es die folgenden Schritte umfasst:

- Inkontaktbringen eines Moläquivalents eines Säurechlorids der Formel (II): ACOCl, wobei A wie oben definiert ist, mit zwei Moläquivalenten eines silylierten Phosphits der Formel $P[OSiMe_3]_2[OMe]$,
- eine Hydrolyse der im vorhergehenden Schritt erhaltenen Verbindungen.

5. Verfahren zur Herstellung von Derivaten von Bisphosphonat der folgenden Formel (I):

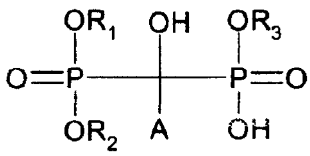

(I),

wo $R_1$ für einen Alkyl-, Aryl-, Acyloxyalky- oder Heterocyclusrest steht und $R_2$ und $R_3$ für ein Wasserstoffatom stehen, A und die eventuellen Substituenten der zuvor genannten Reste wie in Anspruch 1 definiert sind, **dadurch gekennzeichnet, dass** es die folgenden Schritte umfasst:

- Inkontaktbringen wenigstens eines Säurehalogenids der Formel (II): ACOX, wie in Anspruch 1 definiert, mit wenigstens einem silylierten Phosphit der Formel (IV), wie in Anspruch 1 definiert, in der x den Wert 2 besitzt, anschließend Inkontaktbringen des so erhaltenen Produkts mit wenigstens einem silylierten Phosphit der Formel (IV), wie in Anspruch 1 definiert, in der x den Wert 3 besitzt,
- eine Hydrolyse der im vorhergehenden Schritt erhaltenen Verbindungen.

6. Verfahren gemäß einem der vorhergehenden Ansprüche, **dadurch gekennzeichnet, dass** A für einen Rest der Formel $-(CH_2)_n$-R4 steht, in der $R_4$ für einen C1-6 Alkylrest, einen Heterocyclusrest, einen Phenylrest oder einen Rest der Formel $-NR_5R_6$ steht, in der $R_5$ und $R_6$ identisch oder verschieden sind und für ein Wasserstoffatom oder einen C1-6 Alkylrest stehen, in der vorteilhaft n eine ganze Zahl von 1 bis einschließlich 6, vorzugsweise von 1 bis 3, ist.

7. Verfahren gemäß einem der vorhergehenden Ansprüche, **dadurch gekennzeichnet, dass** A für einen Rest steht, der ausgewählt ist aus:

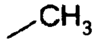 ; 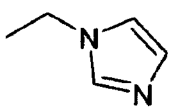 ; 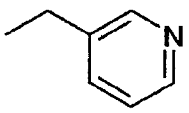 ; 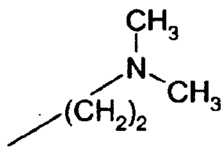 ;

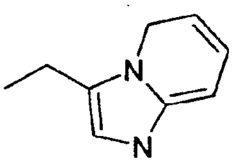 ; 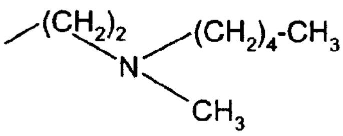 ; 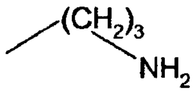 ;

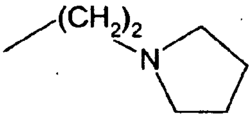 ; 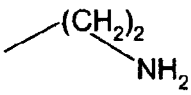 ; und 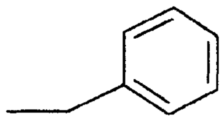 .

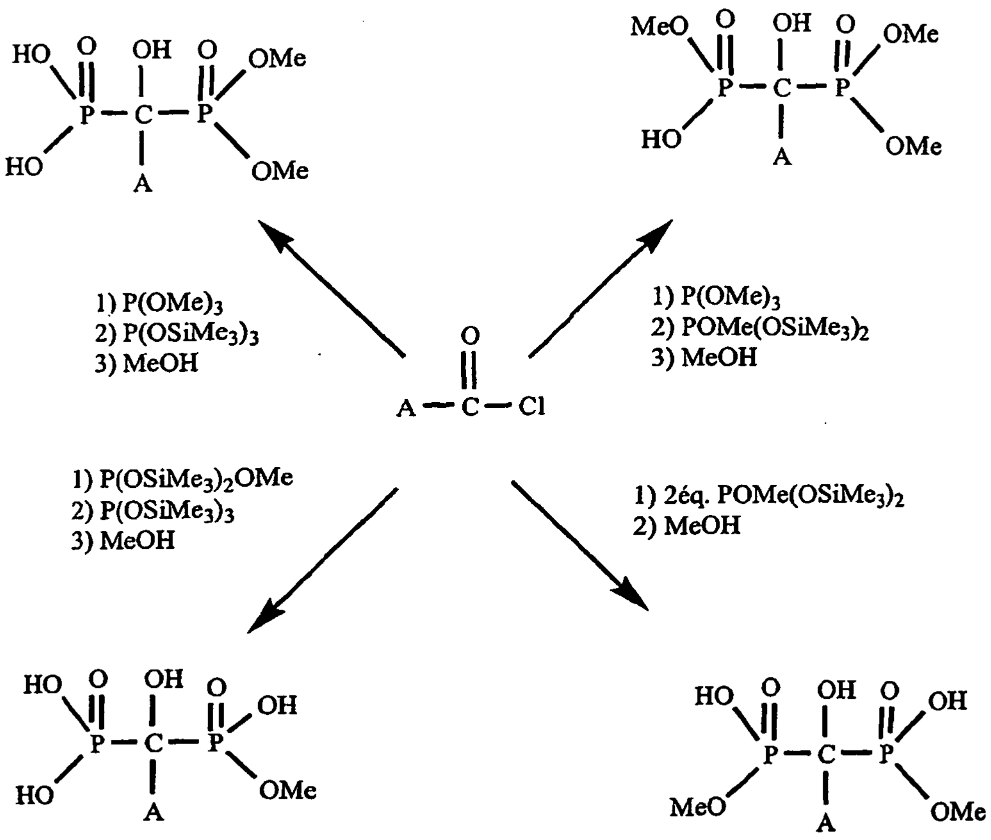
1) $P(OMe)_3$
2) $P(OSiMe_3)_3$
3) MeOH
1) $P(OMe)_3$
2) $POMe(OSiMe_3)_2$
3) MeOH
1) $P(OSiMe_3)_2OMe$
2) $P(OSiMe_3)_3$
3) MeOH
1) 2éq. $POMe(OSiMe_3)_2$
2) MeOH

FIGURE 1

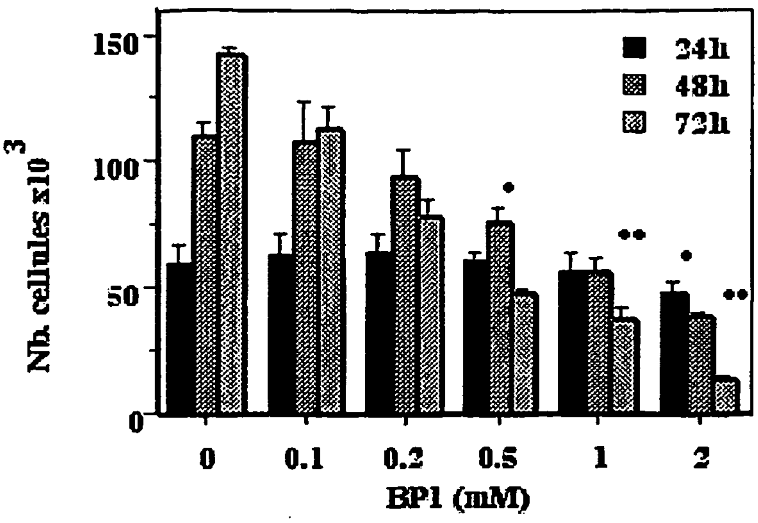


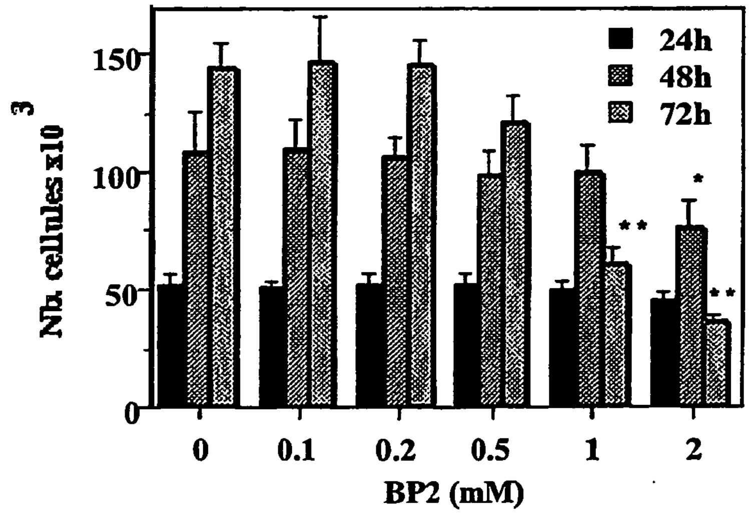


FIGURES 2 ET 3

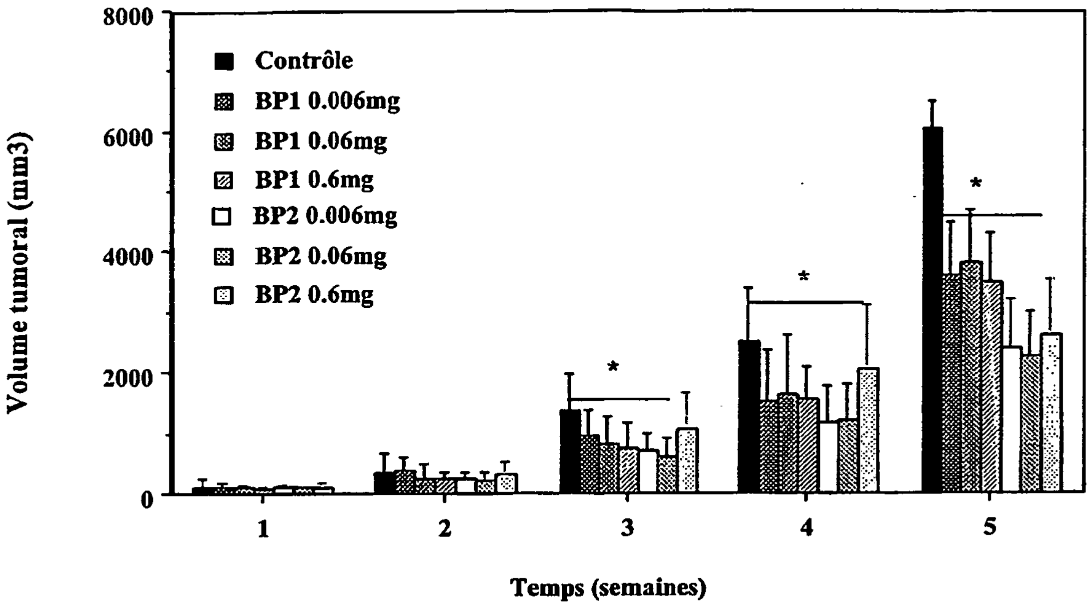
8000
6000
4000
2000
0
Volume tumoral (mm3)
Contrôle
BP1 0.006mg
BP1 0.06mg
BP1 0.6mg
BP2 0.006mg
BP2 0.06mg
BP2 0.6mg
*
*
*
1
2
3
4
5
Temps (semaines)

FIGURE 4

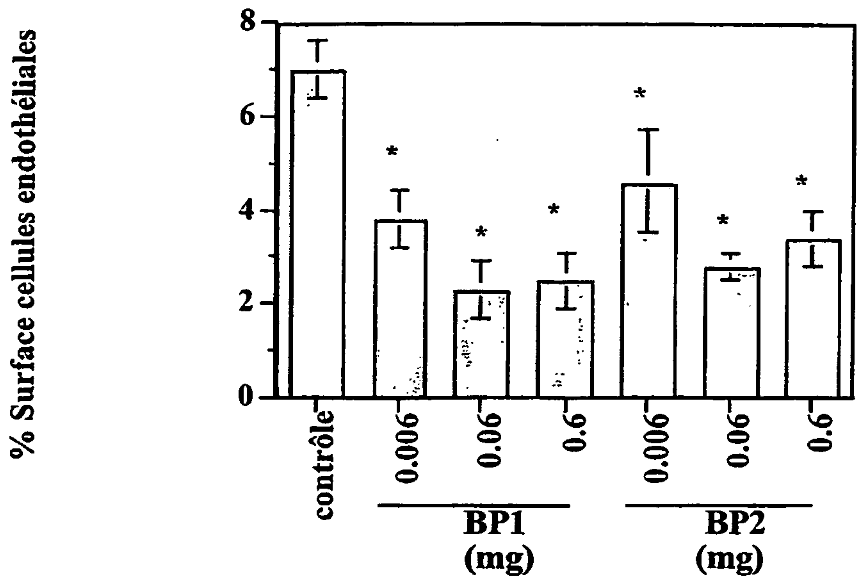
% Surface cellules endothéliales
8
6
4
2
0
contrôle
0.006
0.06
0.6
0.006
0.06
0.6
BP1
(mg)
BP2
(mg)
*

FIGURE 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 8441 M
- US 4705651 A
- GB 2312165 A
- WO 9749711 A
- US 4473560 A
- WO 9704785 A
- EP 0537008 A
- FR 2669348


### Littérature non-brevet citée dans la description


- **Y. Leroux et al.** *Phosphorus, Sulfur and Silicon,* 1991, vol. 63, 181
- **Adami S. ; Zamberlan N.** Adverse effects of bisphosphonates. A comparative review. *Drug Saf.,* 1996, vol. 14, 158-170
- **Boissier S. ; Ferrerras M. ; Peyruchaud O. ; Magnetto S. ; Ebetino F. ; Colombel, Delmas P. ; Delaissé JM ; Clézardin P.** Bisphosphonates inhibit breast and cancer prostate carcinoma cell invasion, an early event in the formation of bone metastases. *Cancer Res.,* 2000, vol. 60, 2949-2954
- **Boissier S. ; Magnetto S. ; Frappart L. ; Cuzin B. ; Ebetino F. ; Delmas P. ; Clézardin P.** Bisphosphonates inhibit prostate and breast carcinoma cell adhesion to unmineralized and mineralized bone extracellular matrices. *Cancer Res.,* 1997, vol. 57, 3890-3894
- **Diel I. ; Solomayer E. ; Bastert G.** Bisphosphonates and the prevention of metastasis: first evidences from preclinical and clinical studies. *Cancer,* 2000, vol. 88, 3080-3088
- **Ezra A ; Hoffman A ; Breuer E ; Alferiev IS ; Monkkonen J ; El Hanany-Rozen N ; Weiss G ; Stepensky D ; Gati I ; Cohen H.** A peptide prodrug approach for improving bisphosphonate oralabsorption. *J Med Chem,* 2000, vol. 43, 3641-3652
- **Fromigue O. ; Lagneaux L. ; Body J.** Bisphosphonates induce breast cancer cell death in vitro. *J. Bone Miner. Res.,* 2000, vol. 15, 2211-2221
- **Hiraga T. ; Williams P. ; Mundy G. ; Yoneda T.** The bisphosphonate ibandronate promotes apoptosis in MDA MB 231 human breast cancer cells in bone metastases. *Cancer Res.,* 2001, vol. 61, 4418-4424
- **Ichinose Y. ; Migita K. ; Nakashima A. ; Kawakami A. ; Aoyagi T. ; Eguchi K.** Effects of bisphosphonate on the release of MMP-2 from cultured human osteoblasts. *Tohoku J. Exp. Med.,* 2000, vol. 192, 111-118
- **Jagdev S. ; Coleman R. ; Shipman C. ; Rostami H. ; Croucher P.** The bisphophonate, zoledronic acid, induces apoptosis of breast cancer cells : evidence for synergy with paclitaxel. *Br., J. Cancer,* 2001, vol. 84, 1126-1134
- **Lee M. ; Fong E. ; Singer R. ; Guenette R.** Bisphosphonate treatment inhibits the growth of prostate cancer cells. *Cancer Res.,* 2001, vol. 61, 2602-2608
- **Lin JH.** Bisphosphonates: a review of their pharmacokinetic properties. *Bone,* 1996, vol. 18, 75-85
- **Lipton A.** Bisphosphonates and breast carcinoma: present and future. *Cancer,* 2000, vol. 88, 3033-3037
- **Luckman SP ; Hugues D. ; Coxon F. ; Graham R. ; Russell G. ; Rogers M.** Nitrogen-containing phosphonates inhibit the mevalonate pathway and prevent post-translational prenylation of GTP-binding proteins, including Ras. *J. Bone Miner. Res.,* 1998, vol. 13, 581-589
- **Mincey B. ; Moraghan T. ; Perez E.** Prevention and treatment of osteoporosis in women with breast cancer. *Mayo Clin. Proc.,* 2000, vol. 75, 821-829
- **Mondelo N. ; Peluffo V. ; Parma. ; Cointry G. ; Capozza R. ; Ferretti J. ; Piccini E. ; Montuori E.** Preclinical toxicology of bisphosphonates. *Medicina (B Aires),* 1997, vol. 57, 93-100
- **Ogihara T. ; Kikuchi Y. ; Imai Y. ; Ohsaka A. ; Isaka M. ; Oka Y.** Acute lymphoblastic leukemia accompagnied by severe hyper calcemia; successful treatment with bisphosphonate. *Rinsho Ketsueki,* 1995, vol. 36, 29-34
- **Pade V. ; Stavchansky S.** Estimation of the relative contribution of the transcellular and paracellular pathway to the transport of passively absorbed drugs in the Caco-2 cell culture model. *Pharm. Res.,* 1997, vol. 9, 1210-1215

- **Patashnik S. ; Rabinovich L. ; Golomb G.** Preparation and evaluation of chitosan microspheres containing bisphosphonates. *J. Drug Target,* 1997, vol. 4, 371-380
- **Perez-Atayde A. ; Sallan S. ; Tedrow U. ; Connors S. ; Allred E. ; Folkman J.** Spectrum of tumor angiogenesis in the bone marrow of children with acute lymphoblastic leukemia. *Am. J. Pathol.,* 1997, vol. 150, 815-821
- **Ruifrok P. ; Mol W.** Paracellular transport of inorganic and organic ions across the rat ileum. *Biochem. Pharmacol.,* 1983, vol. 32, 637-640
- **Senaratne SG. ; Pirianov G. ; Mansi JL. ; Amett TR. ; Colston KW.** Bisphosphonates induce apoptosis in human breast cancer cell lines. *Br. J. Cancer,* 2000, vol. 82, 1459-1468
- **Shipman C. ; Croucher P. ; Russell R. ; Helfrich M. ; Rogers M.** The bisphosphonate incadronate (YM 175) causes apoptosis of human myeloma cells in vitro by inhibiting the mevalonate pathway. *Cancer Res.,* 1998, vol. 58, 5294-5297
- **Shipman C. ; Rogers M. ; Apperley J. ; Russell R. ; Croucher P.** Bisphosphonates induce apoptosis in human myeloma cell lines: a novel anti-tumor activity. *Br. J. Haematol.,* 1997, vol. 98, 665-672
- **Shipman C. ; Vanderkerken K. ; Rogers M. ; Lippitt J. ; Asosingh K. ; Hughes D. ; Van Camp B. ; Russel G. ; Croucher P.** The potent bisphosphonate ibandronate does not induce myeloma cell apoptosis in a murine model of established multiple myeloma. *Br. J. Haematol.,* 2000, vol. 111, 283-286
- **Takagi M. ; Takahiashi K. ; Maruyama T. ; Kaneko K. ; Obinata K. ; Tadokoro R. ; Kastumata Miura Y. ; Fujita H. ; Ishimoto K. ; Yabuta K.** Acute lymphoblastic leukemia accompagnied by severe hypercalcemia : successful treatment including aminohydroxypropylidene bisphosphonate (pamidronate disodium). *Pediatr. Hematol. Oncol.,* 1998, vol. 15, 283-286
- **Teronen O. ; Konttinen Y. ; Lindqvist C. ; Salo T. ; Ingman T. ; Lauhio A. ; Ding Y. ; Santavirta S. ; Valleala H. ; Sorsa T.** Inhibition of matrix metalloproteinase-1 by dichloromethylene bisphosphonate (clodronate). *Calcif. Tissue Int.,* 1997, vol. 61, 59-61
- **Van der Pluijm G. ; Vloedgraven H. ; Van Beek E. ; Van der Wee-Pals L. ; Lowik C. ; Papapoulos S.** Bisphosphonates inhibit the adhesion of breast cancer cells to bone matrices in vitro. *J. Clin. Invest.,* 1996, vol. 98, 698-705
- **Van der Pluijm G. ; Sijmons B. ; Vloedgraven H. ; Deckers M. ; Papapoulos S. ; Lowik C.** Monitoring metastatic behavior of human tumor cells in mice with species-specific polymerase chain reaction : elevated expression of angiogenesis and bone resorption stimulators by breast cancer in bone metastases. *J. Bone Miner. Res.,* 2001, vol. 16, 1077-1091
- **Ylitalo R. ; Monkkonen J. ; Yla-Herttuala S.** Effectes of liposome encapsulated bisphosphonates on acetylated LDL metabolism, lipid accumulation and viability of phagocyting cells. *Life Sci.,* 1998, vol. 62, 413-422
- **Yoneda T. ; Michigami T. ; Yi B. ; Williams P.J. ; Niewolna M. ; Hiraga T.** Actions of bisphosphonate on bone metastasis in animal models of breast carcinoma. *Cancer,* 2000, vol. 15, 2979-2988